# EUROPEAN PATENT SPECIFICATION

(11) **EP 3 707 508 B1**
(45) Date of publication and mention of the grant of the patent: **15.04.2026**
(21) Application number: 18796477.0
(22) Date of filing: 08.11.2018
(51) Int. Cl.: G01N 33/543, G01N 24/08, G01R 33/16

(54) **METHOD FOR DETECTION OF AN ANALYTE**
VERFAHREN ZUR DETEKTION EINES ANALYTEN
PROCÉDÉ DE DÉTECTION D'UN ANALYTE

(30) Priority: 10.11.2017 EP 17382758
(43) Date of publication of application: 16.09.2020
(73) Proprietor: Fundación Imdea Nanociencia, 28049 Madrid (ES)
(72) Inventor: CABRERA CARRASCO, David, E-28049 Madrid (ES); AIRES TRAPOTE, Antonio, E-28049 Madrid (ES); ARTÉS IBAÑEZ, Emilio José, E-28049 Madrid (ES); CAMARERO DE DIEGO, Julio, E-28049 Madrid (ES); LÓPEZ CORTAJARENA, Aitziber, E-28049 Madrid (ES); TERÁN GARCINUÑO, Francisco José, E-28049 Madrid (ES)
(74) Representative: ABG Intellectual Property Law, S.L.
(86) International application number: PCT/EP2018/080662
(87) International publication number: WO 2019/092131

(56) References cited:
- WO-A1-03/019188
- WO-A1-2013/080145
- WO-A1-2017/062821
- ANDREA FORNARA ET AL: "Tailored Magnetic Nanoparticles for Direct and Sensitive Detection of Biomolecules in Biological Samples", NANO LETTERS, vol. 8, no. 10, 8 October 2008 (2008-10-08), pages 3423 - 3428, XP055453346, ISSN: 1530-6984, DOI: 10.1021/nl8022498
- GARRAUD NICOLAS ET AL: "Design and validation of magnetic particle spectrometer for characterization of magnetic nanoparticle relaxation dynamics", AIP ADVANCES, AMERICAN INSTITUTE OF PHYSICS, 2 HUNTINGTON QUADRANGLE, MELVILLE, NY 11747, vol. 7, no. 5, 2 March 2017 (2017-03-02), XP012216697, DOI: 10.1063/1.4978003
- SCOTT J. KEMP ET AL: "Monodisperse magnetite nanoparticles with nearly ideal saturation magnetization", RSC ADVANCES, vol. 6, no. 81, 8 August 2016 (2016-08-08), pages 77452 - 77464, XP055453354, DOI: 10.1039/C6RA12072E
- YI-TING CHEN ET AL: "Biosensing Using Magnetic Particle Detection Techniques", SENSORS, vol. 17, no. 12, 10 October 2017 (2017-10-10), pages 2300, XP055453366, DOI: 10.3390/s17102300
- CONNORD V ET AL: "An air-cooled Litz wire coil for measuring the high frequency hysteresis loops of magnetic samples-A useful setup for magnetic hyperthermia applicat", REVIEW OF SCIENTIFIC INSTRUMENTS, AIP, MELVILLE, NY, US, vol. 85, no. 9, 1 January 1901 (1901-01-01), XP012190135, ISSN: 0034-6748, [retrieved on 19010101], DOI: 10.1063/1.4895656

## Description

### Field of the art

The present invention relates to a method for *in vitro* detection and/or quantification of an analyte present in aqueous or biological fluids. The invention also provides a method for measuring the efficacy of a treatment of a disease in a subject, a method of diagnosis of a disease in a subject, as well as an apparatus for carrying out both methods and its uses. In addition, the invention is directed to the use of functionalized magnetic nanoparticles for *in vitro* detection and/or quantification of an analyte in aqueous or biological fluids, for measuring the efficacy of a treatment of a disease in a subject, and for *in vitro* diagnosing a disease.

### State of the Art

During the last 20 years the use of magnetic nanoparticles (MNPs) in biomedicine has been widely explored for diagnostic purposes, in particular as contrast agents for magnetic resonance imaging (MRI) or magnetic particle imaging (MPI), as magnetic transducer or as magnetic separation agents for sensing (cell markers). For the latest application, Weber C, et al. [Scientific and Clinical Applications of Magnetic Carriers] Springer US: Boston, MA, 1997, pp 371- 378] discloses MNPs functionalized with antibodies as magnetic separation agents to decontaminate blood of infectious agents, or to detect protein analytes based on bio-barcode amplification strategy [Nam J-M et al., Science 2003, 301(5641): 1884-1886.]. The magneto-optical detection of biomolecules by magnetic nanoparticles is also reported by Mezger et al. [ACS Nano 2015, 9(7)7374-7382] disclosing the identification of bacteria from urine samples based on padlock probe recognition followed by two cycles of rolling circle amplification (RCA). Wang W. [Scientific Reports 2014, 4:5716] also reports the detection of DNA by a Giant Magneto Resistance (GMR) sensor using MNPs with sizes ranging from 10 to 100 nm. Fornara et al. [Nano Lett., 2008, 8, 3423] describes a high sensitivity magnetic detection method based on changes in the magnetic susceptibility under an alternating magnetic fields. The method has shown a limit of detection of 0.05 µg·mL⁻¹ of an antibody in biological samples without any pretreatment. However, the disclosed method requires time of measurement of few tens of minutes and sample volumes of the order of 0.5 mL. Besides, Gandhi S. [Nano Letters 2016, 16(6): 3668-3674] discloses the detection of cancer specific proteases by a magnetic particle spectrometer (MPS) relying only on the magnetic relaxation mechanisms of functionalized nanoparticles. However, in the methods reported in the state of the art the extraction of the analyte from their natural media is required for detection. Garraud, Nicolas, et al. (AIP Advances, vol. 7, no. 5, American Institute of Physics, 2 Huntington Quadrangle, Melville, NY 11747, 2 March 2017) describes a magnetic particle spectrometer. Connord, V. et al. Review of Scientific Instruments, vol. 85, no. 9, AIP, Melville, NY, USA, 1 January 1901 was a review article directed to air-cooled Litz wire coils for measuring the high frequency hysteresis loops of magnetic samples for magnetic hyperthermia applications. However, the calorimeter losses in the apparatus of Connord, V. et al. are large, thus, preventing to perform any correct calorimetric measurements inside said set up.

Therefore, there is a need in the art for easy and simple methods for magnetic detection of biomolecules in their natural biological fluids.

### Brief description of the invention

The authors have discovered that surprisingly the specific interaction of functionalised magnetic nanoparticles with an analyte produces a variation in the dynamical magnetisation signal of said functionalized magnetic nanoparticles, which can be detected by magnetisation measurements under alternating magnetic fields. Thus, the present invention provides a method for *in vitro* detection and/or quantification of an analyte in aqueous or biological fluids based on determining the variation of the dynamical magnetisation signal of functionalized magnetic nanoparticles after their specific interaction with an analyte. Thus, one aspect the invention relates to a method for *in vitro* detection and/or quantification of an analyte in aqueous or biological fluids comprising:
a) providing functionalized magnetic nanoparticles, wherein each functionalized magnetic nanoparticle comprises a magnetic nanoparticle and a recognition ligand, wherein the magnetic nanoparticle has an average size of from 1 to 100 nm and a saturation magnetisation comprised between 20 and 300 emu/g, and wherein the recognition ligand is linked to said magnetic nanoparticle,
b) incubating the functionalized magnetic nanoparticles of step a) with an aqueous or biological fluid containing the analyte in conditions suitable for producing the binding of said functionalized magnetic nanoparticles to the analyte,
c) measuring the dynamical magnetisation signal of the functionalized magnetic nanoparticles in the aqueous or biological fluid of step b) containing the analyte under an alternating magnetic field, and
d) comparing the dynamical magnetisation signal of the aqueous or biological fluid measured in step c) with a reference value to detect and/or quantify the presence of the analyte in the aqueous o biological fluid; and
wherein the reference value in step d) is that resulting from measuring the dynamical magnetisation signal of the functionalized magnetic nanoparticle of step a) of a reference sample containing the aqueous or biological fluid without the analyte under an alternating magnetic field;
wherein the dynamical magnetisation signals of steps c) and d) are measured with an apparatus comprising an AC magnetometer; and
wherein the dynamical magnetisation signals are measured as follows:
   I. by measuring the induced voltage signals as a function of time under alternating magnetic fields for:
      - the functionalized magnetic nanoparticles in the aqueous or biological fluid of step b) containing the analyte under an alternating magnetic field; and/or for
      - the functionalized magnetic nanoparticle of step a) of a reference sample containing the aqueous or biological fluid without the analyte under an alternating magnetic field; and
   II. by obtaining the hysteresis loop from said induced voltage signals as a function of time for:
      - the functionalized magnetic nanoparticles in the aqueous or biological fluid of step b) containing the analyte under an alternating magnetic field; and/or for
      - the functionalized magnetic nanoparticle of step a) of a reference sample containing the aqueous or biological fluid without the analyte under an alternating magnetic field; and
wherein the comparison of the dynamical magnetisation signal of step d) is performed by comparing the hysteresis loops; preferably by comparing the values of the hysteresis loop parameters obtained from the hysteresis loops; and wherein the dynamical magnetisation signal is measured under alternating magnetic fields at a single resonant frequency.

In a second aspect the invention relates to the use of functionalized magnetic nanoparticles for *in vitro* detection and/or quantification of an analyte in aqueous or biological fluids, wherein each functionalized magnetic nanoparticle comprises a magnetic nanoparticle and a recognition ligand, wherein the magnetic nanoparticle has an average size of from 1 to 100 nm and a saturation magnetisation comprised between 20 and 300 emu/g, wherein the recognition ligand is linked to said magnetic nanoparticle, and wherein said analyte is detected in aqueous or biological fluids according to the method above disclosed.

In a third aspect the invention relates to a method for measuring the efficacy of a treatment of a disease in a subject, comprising:
a) providing functionalized magnetic nanoparticles, wherein each functionalized magnetic nanoparticle comprises a magnetic nanoparticle and a recognition ligand, wherein the magnetic nanoparticle has an average size of from 1 to 100 nm and a saturation magnetisation comprised between 20 and 300 emu/g, and wherein the recognition ligand is linked to said magnetic nanoparticle,
b) incubating the functionalized magnetic nanoparticles of step a) with an aqueous or biological fluid containing an analyte from the subject in conditions suitable for producing the binding of said functionalized magnetic nanoparticles to the analyte,
c) measuring the dynamical magnetisation signal of the functionalized magnetic nanoparticles in the aqueous or biological fluid from the subject of step b) under an alternating magnetic field, and measuring the dynamical magnetisation signal of a reference sample under an alternating magnetic field, wherein said reference sample is obtained from the same subject at an earlier time of point of the disease or prior to the disease, and
d) comparing the dynamical magnetisation signal of the aqueous or biological fluid of the treated subject and that of the reference sample measured in step c), wherein a change of the dynamical magnetic signal of the treated subject with respect to the dynamical magnetic signal of the reference sample is indicative of the efficacy of a treatment of a disease in a subject; and

wherein the dynamical magnetisation signals of step c) are measured with an apparatus comprising an AC magnetometer; and
wherein the dynamical magnetisation signal is measured as follows:
   I. by measuring the induced voltage signals as a function of time under alternating magnetic fields for:
      - the functionalized magnetic nanoparticles in the aqueous or biological fluid of step b) containing the analyte under an alternating magnetic field; and/or for
      - the functionalized magnetic nanoparticle of step a) of a reference sample containing the aqueous or biological fluid without the analyte under an alternating magnetic field; and
   II. by obtaining the hysteresis loop from said induced voltage signals as a function of time for:
      - the functionalized magnetic nanoparticles in the aqueous or biological fluid of step b) containing the analyte under an alternating magnetic field; and/or for
      - the functionalized magnetic nanoparticle of step a) of a reference sample containing the aqueous or biological fluid without the analyte under an alternating magnetic field;
wherein the comparison of the dynamical magnetisation signal of step d) is performed by comparing the hysteresis loops; preferably by comparing the values of the hysteresis loop parameters obtained from the hysteresis loops; and
wherein the dynamical magnetisation signal is measured under alternating magnetic fields at a single resonant frequency.

In a fourth aspect the invention relates to the use of functionalized magnetic nanoparticles for measuring the efficacy of a treatment of a disease in a subject, wherein each functionalized magnetic nanoparticle comprises a magnetic nanoparticle and a recognition ligand, wherein the magnetic nanoparticle has an average size of from 1 to 100 nm and a saturation magnetisation comprised between 20 and 300 emu/g, and wherein the recognition ligand is linked to said magnetic nanoparticle, and wherein the efficacy of said treatment is measured according to the method above disclosed for measuring the efficacy of a treatment of a disease. The invention also relates to the use of functionalized magnetic nanoparticles for measuring the efficacy of a treatment of a disease in a subject, wherein the disease is selected from cancer, autoimmune diseases, neurodegenerative diseases, cardiovascular diseases, inflammatory diseases, and endocrine diseases, among others.

In other aspect the invention relates to an apparatus designed for carrying out the method of the invention for *in vitro* detection and/or quantification of an analyte in aqueous or biological fluids or for carrying out the method for measuring the efficacy of a treatment of a disease in a subject comprising an AC magnetometer for measuring the dynamical magnetisation signal of functionalized magnetic nanoparticles comprising:
a) an AC magnetic field generator configured to magnetically excite the functionalized magnetic nanoparticles, said AC magnetic field generator comprising a Litz wire coil as an excitation coil, wherein the AC magnetic field generator is part of a LCR circuit allowing to resonantly inject an AC current of a single resonant frequency to the Litz wire coil generating an AC magnetic field wherein the single resonant frequency is within the frequency range from 10 Hz to 1 MHz,
b) a magnetic flux detector comprising two counterwise wounded pick-up coils connected in series and mounted inside the excitation coil, wherein the two pick-up coils have the same turns and dimensions, and
c) a voltage reader,

wherein the voltage reader monitors the voltage signal of the pick-up coils of the magnetic flux; and
wherein the excitation coil is immersed into a dielectric liquid flow that circulates across a heat exchanger for thermalization at room temperature.

Another aspect of the invention relates to a method of diagnosis of a disease in a subject comprising the following steps:
a) providing functionalized magnetic nanoparticles, wherein each functionalized magnetic nanoparticle comprises a magnetic nanoparticle and a recognition ligand, wherein the magnetic nanoparticle has an average size of from 1 to 100 nm and a saturation magnetisation comprised between 20 and 300 emu/g, and wherein the recognition ligand is linked to said magnetic nanoparticle,
b) incubating the functionalized magnetic nanoparticles of step a) with an aqueous or biological fluid from the subject in conditions suitable for producing the binding of said functionalized magnetic nanoparticles to an analyte, wherein said analyte is a biomarker of the disease to be diagnosed,
c) measuring the dynamical magnetisation signal of the functionalized magnetic nanoparticles in the aqueous or biological fluid from the subject of step b) under an alternating magnetic field, and
d) comparing the dynamical magnetisation signal of the aqueous or biological fluid of the subject measured in step c) with a reference value indicative of the disease to be diagnosed; and

wherein the dynamical magnetisation signals are measured with an apparatus comprising an AC magnetometer; and
wherein the dynamical magnetisation signal is measured as follows:
   I. by measuring the induced voltage signals as a function of time under alternating magnetic fields for:
      - the functionalized magnetic nanoparticles in the aqueous or biological fluid of step b) containing the analyte under an alternating magnetic field; and/or for
      - the functionalized magnetic nanoparticle of step a) of a reference sample containing the aqueous or biological fluid without the analyte under an alternating magnetic field; and
   II. by obtaining the hysteresis loop from said induced voltage signals as a function of time for
      - the functionalized magnetic nanoparticles in the aqueous or biological fluid of step b) containing the analyte under an alternating magnetic field; and/or for
      - the functionalized magnetic nanoparticle of step a) of a reference sample containing the aqueous or biological fluid without the analyte under an alternating magnetic field; and
      wherein the comparison of the dynamical magnetisation signal of step d) is performed by comparing the hysteresis loops; preferably by comparing the values of the hysteresis loop parameters obtained from the hysteresis loops; and
wherein the dynamical magnetisation signal is measured under alternating magnetic fields at a single resonant frequency.

Another aspect of the invention relates to the apparatus designed for carrying out the method defined according to claim 12, as defined in claim 13.

Another aspect of the invention relates to the use of the apparatus of the invention, as defined in claim 14, in the method of the invention for *in vitro* detection of an analyte in aqueous or biological fluids or in the method of the invention for measuring the efficacy of a treatment of a disease in a subject or in the method of the invention of diagnosis of a disease, for measuring the dynamical magnetisation signal of the functionalized magnetic nanoparticles dispersed into the aqueous or biological fluids.

Another aspect of the invention relates to the *in vitro* use of functionalized magnetic nanoparticles for diagnosing a disease, wherein each functionalised magnetic nanoparticles comprises a magnetic nanoparticle and a recognition ligand, wherein the magnetic nanoparticle has an average size of from about 1 to about 100 nm and a saturation magnetisation comprised between 20 and 300 emu/g, wherein the recognition ligand is linked to said magnetic nanoparticle, and wherein the disease is diagnosed by *in vitro* detection and/or quantification of an analyte in aqueous or biological fluids according to the method for *in vitro* detection and/or quantification of an analyte in aqueous or biological fluids above disclosed. The invention also relates to the use of functionalized magnetic nanoparticles for diagnosing a disease in a subject, wherein the disease is selected cancer, autoimmune diseases, neurodegenerative diseases, cardiovascular diseases, inflammatory diseases, and endocrine diseases, among others.

### Figures

Figure 1: General Scheme of the method of the invention.
Figure 2: A) Analyte concentration dependence of the AC hysteresis loops of DMSA-MNP-GST-MEEVF upon the addition of increasing concentrations of VFP_{d}-TPR2-MMY in PBS at 1 mg Fe/mL, 100 kHz and 35 mT ; B) Analyte concentration dependence of the area of the AC hysteresis loops from figure 2A.
Figure 3: A) schematic representation of an AC magnetometer; B) schematic representation of the electronic circuit for generating AC magnetic fields.
Figure 4: A) graph showing different induced voltage signals as a function of time; B) AC magnetic hysteresis loop (M) represented versus the magnetic field intensity; and C) comparison of mass normalized AC (105 kHz) and DC magnetisation cycles from MNP dispersed in water at room temperature.
Figure 5: A) binding curve of VFPₘ-TPR2-MMY onto DMSA-MNP-GST-MEEVF, line is fitting based on a 1:1 binding model (y=Bₘₐₓ·x/(K_{d}+x)) to calculate the dissociation constant (K_{d}); B) hysteresis loop of DMSA-MNP-GST-MEEVF at 0 and 4 µM of VFPₘ-TPR2-MMY in PBS.
Figure 6: A) hysteresis loop of PMAO-MNP-GST-MEEVF upon the addition of increasing concentrations of VFPₘ-TPR2-MMY in PBS; B) Analyte concentration dependence of the area extracted from Fig. 6A.
Figure 7: A) Analyte concentration dependence of the area extracted from AC hysteresis loop of DMSA-MNP-GST-AP-Biotin upon the addition of increasing concentrations of avidin in PBS; B) Analyte concentration dependence of the area extracted from hysteresis loop of DMSA-MNP-GST-AP-Biotin upon the addition of increasing concentrations of avidin in human plasma; and C) analyte concentration dependence of the area extracted from hysteresis loop of PMAO-MNP-GST-AP-Biotin upon the addition of increasing concentrations of avidin in PBS.

### Detailed description of the invention

The present invention provides a method for *in vitro* detection and/or quantification of an analyte in aqueous or biological fluids comprising:
a) providing functionalized magnetic nanoparticles, wherein each functionalized magnetic nanoparticle comprises a magnetic nanoparticle and a recognition ligand, wherein the magnetic nanoparticle has an average size of from 1 to 100 nm and a saturation magnetisation comprised between 20 and 300 emu/g, and wherein the recognition ligand is linked to said magnetic nanoparticle,
b) incubating the functionalized magnetic nanoparticles of step a) with an aqueous or biological fluid containing the analyte in conditions suitable for producing the binding of said functionalized magnetic nanoparticles to the analyte,
c) measuring the dynamical magnetisation signal of the functionalized nanoparticles dispersed in an aqueous or biological fluid of step b) containing the analyte under an alternating magnetic field, and
d) comparing the dynamical magnetisation signal of the aqueous or biological fluid measured in step c) with a reference value to detect and/or quantify the presence of the analyte in the aqueous o biological fluid; and
wherein the reference value in step d) is that resulting from measuring the dynamical magnetisation signal of the functionalized magnetic nanoparticle of step a) of a reference sample containing the aqueous or biological fluid without the analyte under an alternating magnetic field; and
wherein the dynamical magnetisation signals of steps c) and d) are measured with an apparatus comprising an AC magnetometer; and
wherein the dynamical magnetisation signals are measured as follows:
   I. by measuring the induced voltage signals as a function of time under alternating magnetic fields for:
      - the functionalized magnetic nanoparticles in the aqueous or biological fluid of step b) containing the analyte under an alternating magnetic field; and/or for
      - the functionalized magnetic nanoparticle of step a) of a reference sample containing the aqueous or biological fluid without the analyte under an alternating magnetic field; and
   II. by obtaining the hysteresis loop from said induced voltage signals as a function of time for
      - the functionalized magnetic nanoparticles in the aqueous or biological fluid of step b) containing the analyte under an alternating magnetic field; and/or for
      - the functionalized magnetic nanoparticle of step a) of a reference sample containing the aqueous or biological fluid without the analyte under an alternating magnetic field; and

wherein the comparison of the dynamical magnetisation signal of step d) is performed by comparing the hysteresis loops; preferably by comparing the values of the hysteresis loop parameters obtained from the hysteresis loops; and
wherein the dynamical magnetisation signal is measured under alternating magnetic fields at a single resonant frequency.

In the context of the present invention the term "in vitro" or "ex vivo" refers to the performance of experiments or studies outside their normal biological context, in particular out of the animal or human body. In this sense, the methods provided in the present application are not practiced on the human or animal body but out of the animal or human body.

The term "analyte", as used herein, refers to any biomarker molecule without limitation.

The term "biomarker" in the context of the present invention refers to a measurable indicator of some biological state or condition.

Analytes or biomarkers suitable for the method of the invention include any macromolecules or small molecules, as well as certain cells. In a particular embodiment the analyte is selected from drugs, doping agents, proteins, peptides, pseudopeptides, nucleic acids, nucleic acid-protein complexes, mRNA, microRNA, lipids, vesicles, vesicle markers, cancerous cell, amino acids, amino acids derivatives, sugars, alkaloids, glycosides, non-ribosomal peptides, phenazines, natural phenols, polyketide, terpenes, and tetrapyrroles.

The term "doping agents" refers to specific drugs that enhance performance. Examples of doping agents in the context of the present invention include steroids, anabolic steroids, β2-agonists, erythropoietins (EPO, CERA), human chorionic gonadotrophin (hCG), growth hormone (hGH), perfluorochemicals, efaproxiral (RSR13), and stimulants such as amfetamine, dexamfetamine, ecstasy, lisdexamfetamine, methylphenidate, modafinil, pseudoephedrine and ephedrine.

In a particular embodiment the analyte is a small molecule. As used herein, the term "small molecule" refers to an organic compound either synthesized in the laboratory or found in nature. In the context of the present invention, a small molecule is characterized in that it contains several carbon-carbon bonds, and has a molecular weight of less than 1500, although this characterization is not intended to be limiting for the purposes of the present invention. Small molecules suitable for the method of the invention include alkaloids, glycosides, lipids, non-ribosomal peptides, phenazines, natural phenols, polyketide, terpenes and tetrapyrroles.

In a particular embodiment the analyte to be detected and/or quantified by the method of the invention is a monovalent or multivalent analyte. In the context of the present invention the term "monovalent analyte" refers to an analyte having one binding site to be recognized by the recognition ligand of the functionalized magnetic nanoparticle of the method of the invention. In the context of the present invention the term "multivalent analyte" refers to an analyte having more than one binding site to be recognized by the recognition ligand of the functionalized magnetic nanoparticle of the method of the invention. In a preferred embodiment the analyte is a multivalent analyte.

According to step a) functionalised magnetic nanoparticles are provided, wherein each functionalised magnetic nanoparticle comprises a magnetic nanoparticle and a recognition ligand.

The term "magnetic nanoparticle" or "magnetic NP" or "MNP", as used herein, refers to a particle having a diameter ranging from about 1 to about 100 nanometres and a saturation magnetisation (Ms) comprised between 20 and 300 emu/g.

In a preferred embodiment the magnetic nanoparticle has an average particle diameter ranging from 2 to 50 nm, preferably from 4 to 30 nm, preferably from 10 to 25 nm, more preferably 20 nm. The average particle diameter is understood as the average maximum dimension of the magnetic nanoparticles. The particle size of each magnetic nanoparticle may be measured using common techniques of the state of the art, such as microscopy techniques. The diameter values of the magnetic nanoparticles disclosed in the present application were measured by dynamic light scattering (DLS) that provides the hydrodynamic diameter and by transmission electron microscopy (TEM) that provides the size of the metallic core.

In the context of the present invention the magnetic particles may have any shape. Preferably, the magnetic nanoparticles have a cubic or substantially cubic shape. The shape of the magnetic nanoparticle may be assessed by transmission electron microscopy techniques.

The saturation magnetisation (Ms) of the magnetic nanoparticles is comprised between 20 and 300 emu/g, preferably from 50 to 250 emu/g, even more preferably between 75 and 200 emu/g, even more preferably of 100 emu/g. The saturation magnetisation (Ms) of the magnetic nanoparticle is measured by using standard techniques, such as vibrating sample magnetometry (VSM) or superconducting quantum interference device (SQUID).

The magnetic nanoparticle of the method of the invention may be a metal oxide, preferably Fe, Co or Ni metal oxide. In a particular embodiment, the magnetic nanoparticle is a metal oxide selected from Fe, Co or Ni metal oxide selected from gamma-Fe₂O₃, Fe₃O₄, CoO, Co₃O₄, NiO; a stoichiometric ferrite selected from MnFe₂O₄, CoFe₂O₄, ZnFe₂O₄, NiFe₂O₄, MgFe₂O₄, SrFe₁₂O₁₉ and BaFe₁₂O₁₉; a nonstoichiometric ferrite selected from Fe₃₋ₓMₓO₄, wherein M is a transition element selected from Cr, Mn, Co, Ni and Zn being x >1, MnₐZn₍₁₋ₐ₎Fe₂O₄ and NiₐZn₍₁₋ₐ₎Fe₂O₄ being a<1 and mixtures thereof. In a preferred embodiment the magnetic nanoparticle is a metal oxide selected from gamma-Fe₂O₃ (maghemite), Fe₃O₄(magnetite), CoO, Co₃O₄, and NiO.

In another particular embodiment, the magnetic nanoparticle is based on stoichiometric ferrites, non-stoichiometric ferrites or doped ferrites.

In another preferred embodiment, the magnetic nanoparticle is based on ferrites having the general formula MFe₂O₄, wherein M represents a metal selected from: Co, Ni, Mg, Zn, Sr, or Mn. In another preferred embodiment the magnetic nanoparticle is a stoichiometric ferrite selected from MnFe₂O₄, CoFe₂O₄, ZnFe₂O₄, NiFe₂O₄, MgFe₂O₄, SrFe₁₂O₁₉ or BaFe₁₂O₁₉. In another preferred embodiment the magnetic nanoparticle is a nonstoichiometric ferrite selected from Fe₃₋ₓMₓO₄ wherein M is a transition element selected from Cr, Mn, Co, Ni and Zn being x >1, MnₐZn₍₁₋ₐ₎Fe₂O₄ and NiₐZn₍₁₋ₐ₎Fe₂O₄ being a<1.

In another preferred embodiment the magnetic nanoparticle is based on non-stoichiometric ferrites selected from Fe₃₋ₓMₓO₄ wherein M is a transition elements selected from Cr, Mn, Co, Ni and Zn; Mn Fe₂O₄, MnₐZn₍₁₋ₐ₎Fe₂O₄ and NiₐZn₍₁₋ₐ₎Fe₂O₄ being a<1. In a more preferred embodiment, the magnetic nanoparticle is based on Zn_{0.4}Fe_{2.6}O₄.

In the context of the present invention the term "recognition ligand", "targeting agent", or "receptor" refers to molecules or ligands that specifically interact with and bind to analyte. The receptors may be also a fragment or a variant of any other molecule that specifically binds the analyte. In a preferred embodiment, the recognition ligand is a monovalent recognition ligand or a multivalent recognition ligand. The term "multivalent recognition ligand" in the context of the present invention refers to a molecule that is linked to the magnetic nanoparticle, and that presents more than one binding site to recognize a specific ligand. The term "monovalent recognition ligand" in the context of the present invention refers to a molecule that is linked to the magnetic nanoparticle, and that presents one binding site to recognize an specific ligand of the analyte. In a more preferred embodiment the recognition ligand is a monovalent recognition ligand.

In a particular embodiment the analyte to be detected and/or quantified is either a monovalent or a multivalent analyte and the recognition ligand onto the functionalised magnetic nanoparticle of the method of the invention is either a multivalent recognition ligand or a monovalent recognition ligand. In a particular embodiment the recognition ligand onto the functionalised magnetic nanoparticle of the method of the invention is a multivalent recognition ligand, and the analyte to be detected and/or quantified is either a monovalent or a multivalent analyte. In a preferred embodiment the recognition ligand onto the functionalised magnetic nanoparticle of the method of the invention is a monovalent recognition ligand, and the analyte to be detected and/or quantified is either a monovalent or a multivalent analyte.

In a particular embodiment the recognition ligand is selected from a carbohydrate, a peptide, a pseudopeptide, a peptoid, a protein, an antibody, an aptamer, a DNA probe, a RNA probe, a peptide nucleic acid (PNA), and combinations thereof.

In a particular embodiment the recognition ligand of the functionalised magnetic particle of the method of the invention is a peptide. As used herein, the term "peptide" refers to a short chain of amino acid monomers linked by peptide bonds. The peptide will comprise at least 2 amino acids, at least 3 amino acids, at least 4 amino acids, at least 5 amino acids, at least 10 amino acids, at least 15 amino acids, at least 20 amino acids, at least 30 amino acids, at least 40 amino acids, at least 50 amino acids, at least 60 amino acids, or at least 70 amino acids. Suitable for the purposes of this invention are peptides with, among others, capacity to penetrate a cell, to provoke signalling, or to bind to a target.

In a preferred embodiment, the peptide is selected from the group consisting of a cell-penetrating peptide, a signalling peptide and a target binding peptide.

As used herein, the term "target binding peptide" refers to a peptide comprising a target binding region. Amino acid sequences suitable for binding target molecules include consensus sequences of molecular recognition well known in the art. These include without limitation:
- sequences containing the RGD motif to target integrins, preferably the RGDLXXL (SEQ ID NO: 1) sequence, wherein "X" is any amino acid, such as TTYTASARGDLAHLTTTHARHLP (SEQ ID NO: 2), RGDLATLRQLAQEDGVVGVR (SEQ ID NO: 3), SPRGDLAVLGHKY (SEQ ID NO: 4), CRGDLASLC (SEQ ID NO: 5), etc.;
- the LINK domain from TSG-6 is the preferred sequence to target hyaluronan, but also domains from hyaluronan receptors RHAMM and CD44 can be used;
- the laminin receptor binding peptide [YIGSR (SEQ ID NO: 6)];
- VEGF receptor binding peptide (VRBP) (SEQ ID NO: 7);
- pro-gastrin-releasing peptide (ProGRP) to target gastrin-releasing peptide receptor;
- PHSRN motif from fibronectin to target alpha(5)beta(1) integrin fibronectin receptor (SEQ ID NO: 8);
- NGR that binds aminopeptidase N (CD13).

In another particular embodiment, the recognition ligand of the functionalised magnetic particle of the method of the invention is a pseudopeptide. As used herein, the term "pseudopeptide" refers to analogues of peptide or proteins that mimic the biological activities of natural peptides or proteins. In the context of the present invention pseudopeptides may be peptide analogues obtained by replacing one or more amino acids of the L series with one or more of the corresponding D series, or peptides exhibiting a modification at the level of at least one of the peptide bonds, such as the retro, inverso, retro-inversi, carba and aza bonds. The pseudopeptide will comprise at least 2 amino acids, at least 3 amino acids, at least 4 amino acids, at least 5 amino acids, at least 10 amino acids, at least 15 amino acids, at least 20 amino acids, at least 30 amino acids, at least 40 amino acids, at least 50 amino acids, at least 60 amino acids, or at least 70 amino acids. Suitable for the purposes of this invention are pseudopeptides with, among others, capacity to penetrate a cell, to provoke signalling, to bind to a target.

In a preferred embodiment, pseudopeptides are selected from HB-19, Carfilzomib (PR-171), Oprozomib (PR-047), Delanzomib (CEP-18770), Bortezomib, and Epoxomicin.

The term "peptoid" refers to peptide analogues which have alkyl side chains attached to some of the nitrogen atoms of glycine residues.

In a preferred embodiment, the recognition ligand acting as receptor is an antibody (Ab). The term "antibody" is used herein in the sense of its capacity to bind specifically to an antigen and thus, it refers to a molecule having such capacity. Included within said term are:
- an intact antibody that binds specifically to the target antigen; and
- an antibody fragment that binds specifically to the target antigen.

As used herein, the term "intact antibody" refers to an immunoglobulin molecule capable of specific binding to its cognate target, including a target, such as a carbohydrate, polynucleotide, lipid, polypeptide, etc., through at least one binding recognition site (e.g., antigen binding site), including a site located in the variable region of the immunoglobulin molecule. An antibody includes an antibody of any class, namely IgA, IgD, IgE, IgG (or sub-classes thereof), and IgM, and the antibody need not be of any particular class. In a preferred embodiment, the antibody is an IgG.

As used herein, the term "antibody fragment" refers to functional fragments of antibodies, such as Fab, Fab', F(ab')₂, Fv, single chain (scFv), heavy chain or fragment thereof, light chain or fragment thereof, a domain antibody (DAb) (i.e., the variable domain of an antibody heavy chain (VH domain) or the variable domain of the antibody light chain (VL domain)) or dimers thereof, VH or dimers thereof, VL or dimers thereof, nanobodies (camelid VH), and functional variants thereof, fusion proteins comprising an antibody, or any other modified configuration of the immunoglobulin molecule that comprises an antigen recognition site of a desired specificity. An antibody fragment may refer to an antigen binding fragment. In a preferred embodiment, the antibody fragment is a VH or domain antibody or DAb. In another preferred embodiment, the antibody fragment is a scFv. In another preferred embodiment, the antibody fragment is a nanobody.

Techniques for the preparation and use of the various antibodies are well known in the art. For example, fully human monoclonal antibodies lacking any non-human sequences can be prepared from human immunoglobulin transgenic mice or from phage display libraries.

In a particular embodiment the recognition ligand acting as a receptor is an aptamer. Preferably, the receptor is an aptamer selected from a peptide aptamer and a DNA aptamer. As used herein, the term "peptide aptamer" refers to a short variable peptide domain that is attached at both ends to a protein scaffold, and that binds to a specific target molecule. Aptamers are usually created by selecting them from a large random sequence pool, but natural aptamers also exist in riboswitches. Aptamers can be used for both basic research and clinical purposes as macromolecular drugs. As such, peptide aptamers are proteins that are designed to interfere with other protein interactions inside cells. The variable loop length is typically composed of ten to twenty amino acids, and the scaffold may be any protein which has good solubility and compacity properties. Currently, the bacterial protein Thioredoxin-A is the most used scaffold protein, the variable loop being inserted within the reducing active site, which is a Cys-Gly-Pro-Cys loop (SEQ ID NO: 9) in the wild protein, the two Cys lateral chains being able to form a disulfide bridge. Peptide aptamer selection can be made using different systems, including the yeast two-hybrid system, phage display, mRNA display, ribosome display, bacterial display and yeast display.

The term "DNA aptamer", as used herein, refers to a short strand of DNA that has been engineered through repeated rounds of selection to bind to specific molecular targets, such as small molecules, proteins, nucleic acids, and even cells, tissues and organisms. DNA aptamers are useful in biotechnological and therapeutic applications as they offer molecular recognition properties that rival that of the commonly used biomolecule, antibodies, and elicit little or no immunogenicity in therapeutic applications. The selection of DNA aptamers is well-known in the art using techniques such as systematic evolution of ligands by exponential enrichment (SELEX).

In a particular embodiment the functionalised magnetic particle of the method of the invention is prepared by a process comprising the following steps:
I) activating a magnetic nanoparticle for the immobilisation of a recognition ligand,
II) optionally modifying the recognition ligand for the immobilization on the magnetic nanoparticle, and
III) attaching the recognition ligand of step II to the magnetic nanoparticle of step I.

According to step I) of the above disclosed process the magnetic nanoparticle is activated for the immobilisation of a recognition ligand. In preferred embodiment, the magnetic nanoparticle is coated with an organic hydrophilic compound to be activated for the immobilisation of a recognition ligand. The hydrophilic coating on the magnetic nanoparticle minimizes the non-specific adsorption of molecules from complex biological fluids, maximizing the sensitivity of the detection method. Suitable hydrophilic coatings for the magnetic nanoparticle contain carboxylic acids, PMAO, amine groups, alcohol groups, thiol groups, maleimide, etc.

In a particular embodiment the magnetic nanoparticle is coated with an organic hydrophilic compound containing at least a carboxylic group. The carboxylic groups are used as reactive groups to anchor the recognition ligand onto the MNPs surface in step III, preferentially leading to neutral net charge values of the overall functionalized MNPs when dispersed in double distillate water.

Moreover, the organic coating of the magnetic nanoparticle preserves the colloidal stability in biological fluids (such as blood, blood plasma, urine, saliva, spinal fluid, etc) preventing the non-specific adsorption of biological molecules such as proteins, so that the functionalised magnetic nanoparticle must be stable in aqueous and biological fluids without significant variation of their hydrodynamic size (< 10% variation). Moreover, the functionalization of the magnetic nanoparticles with recognition ligands (i.e. receptor) does not affect the colloidal stability of the magnetic nanoparticles nor the recognition properties of the "analyte" in the biological fluids.

According to step II) of the process disclosed, the recognition ligand is optionally modified for the immobilization on the magnetic nanoparticle.

In a particular embodiment the recognition ligand is an antibody. In a preferred embodiment, when the recognition ligand is an antibody, the antibody is modified according to step II) by the introduction of free thiol groups onto the antibody through the reaction between Traut's reagent or 2-iminothiolane and the amine groups of the antibody. In an antibody molecule, it is possible to distinguish at least two types of amino groups exposed to the medium: (i) the terminal amino groups and (ii) the *ε*-amino moiety of lysine residues, since the terminal amino groups have a pK around 7-8 and *ε-*amino groups of Lys residues have a pK close to 10. At pH values less than 8.0, the Ab amino terminal groups are the most reactive. As the amino terminal moieties are located in the Fab region where antigen recognition takes place, the Ab modification at this pH condition could contribute to a lower activity of the Ab after its functionalization. While at pH values higher than 8.0, *ε*-amino groups of Lys residues are more reactive and as the majority of the lysine residues are located in the Fc portion, the modification should occur preferentially in the Fc portion. Reactions at pH > 8 are particularly preferred in the context of the present invention. Preferably, the targeting agent is then attached covalently to the activated magnetic nanoparticle of step I through the thiol moieties of the magnetic nanoparticle. Hence, the functionalization of the nanoparticle is achieved by the formation of disulfide bonds between the activated nanoparticle and the modified drug, and between the activated nanoparticle and the modified targeting agent.

According to step III) of the above disclosed process, the recognition ligand of step II is attached to the magnetic nanoparticle of step I).

In the context of the present invention, the term "attaching" or "linking" refers to the immobilisation of the recognition ligand onto the magnetic nanoparticle. In a particular embodiment the recognition ligand is attached onto the magnetic nanoparticle by hydrogen bonds, hydrophobic interactions, Van der Waals forces, affinity binding or formation of covalent bonds. Methods of immobilization suitable for the present invention are disclosed for example in Biotechnology & Biotechnological Equipment, 2015, Vol. 29, No. 2, 205-220. In a particular embodiment, the magnetic nanoparticle is linked to the recognition ligand through an amide, ether, thio-ether, or carbamate bonds. The recognition ligand may be immobilized on the surface of the magnetic nanoparticle, by for example adsorption, entrapment, covalent and cross-linking.

In a preferred embodiment, the recognition ligand is linked to the magnetic nanoparticle by hydrophobic or aromatic adsorption, hydrogen bond, electrostatic interactions or covalent bond. Preferably, the recognition ligand is linked to the magnetic particle by a covalent bond. When the recognition ligand is linked to the magnetic particle by covalent bond, the orientation of the recognition ligand on the surface of the magnetic nanoparticle is better controlled.

In a preferred embodiment several recognition ligands are attached to the magnetic nanoparticle.

The term "transducer" in the context of the present invention refers to the functionalized magnetic nanoparticle (F-MNPs) comprising magnetic nanoparticles with one or several recognition ligands attached onto the surface of said magnetic nanoparticles for specifically binding an analyte in aqueous or biological fluids.

According to step b) of the method of the invention for *in vitro* detection and/or quantification of an analyte, the functionalised magnetic nanoparticles (transducer) of step a) are incubated with an aqueous or biological fluid containing the analyte in conditions suitable for producing the binding of said transducer to the analyte. The binding of said transducer to the analyte produces a change of the dynamical magnetic signal of said functionalized magnetic nanoparticles.

In the context of the present invention, the term "binding" refers to the interaction produced between the recognition ligand of the functionalised magnetic nanoparticle and the analyte. In a particular embodiment the recognition ligand of the functionalised magnetic nanoparticle interacts with the analyte through hydrogen bonds, hydrophobic interactions, electrostatic interactions, □ interactions, Van der Waals forces, affinity binding, or formation of covalent bonds.

The term "dynamical magnetic signal" or "dynamical magnetic response" in the context of the present invention is related to the hysteresis loops, such as magnetic hysteresis loops, obtained from the induced voltage signals as a function of time measured under alternating magnetic fields preferably by an AC magnetometer.

Without being bound to a theory in particular, the authors of the present invention have observed that the changes of the dynamical magnetic signal or the changes of the dynamical magnetic response under an alternating magnetic field caused by the binding of an analyte to functionalized magnetic nanoparticles, allow the detection and/or quantification of said analyte in aqueous or biological fluids in the methods of the present invention.

The terms "changes of the dynamical magnetic signal" or "changes of the dynamical magnetic response" in the context of the present invention refer to changes of the hysteresis loops acquired when F-MNPs are subjected to alternating magnetic fields. Those changes in the hysteresis loops are related to changes in the area, the related magnetic parameters (i.e. remanence, coercitivity, maximal magnetisation), and/or the magnetization harmonics. In particular, the dynamical magnetic signal is measured as the induced voltage signals as a function of time under alternating magnetic fields, and consequently, changes in the dynamical magnetic signal are related with (and/or reflected on) changes in the (magnetic) parameters of the hysteresis loops obtained from said induced voltage signals as a function of time, such as changes in the hysteresis loop area. Preferably, the dynamical magnetic signal is measured in an apparatus comprising an AC magnetometer.

In a particular embodiment, the changes in the dynamical magnetic signal are obtained as changes in the hysteresis loop area. In another particular embodiment the changes in the dynamical magnetic signal are obtained as changes in the related magnetic parameters (i.e. remanence, coercitivity, maximal magnetisation) obtained as the hysteresis loop parameters. In another particular embodiment the changes in the dynamical magnetic signal are obtained as changes in the magnetization harmonics.

The conditions suitable for producing the binding of the functionalised magnetic nanoparticles to the analyte are known by the person skilled in the art. In particular, the suitable conditions will depend on the analyte and F-MNP concentration, field conditions, analyte multivalence, as well as on the magnetic properties of the functionalised magnetic particle used.

In a particular embodiment maghemite or cobalt ferrite magnetic nanoparticles having a range size of from 12 to 21 nm coated with dimercapto succinic acid or amphiphilic polimer are incubated 5-60 minutes at 37°C for producing the binding of the functionalised magnetic nanoparticles to the analyte.

According to step c) of the method of the invention, the dynamical magnetization signal of the functionalized magnetic nanoparticles in the aqueous or biological fluid of step b) containing the analyte is measured under an alternating magnetic field.

It is believed that when the analyte to be detected and/or quantified is monovalent, the Brownian relaxation mechanism is altered as consequence of the analyte specific binding that increases the transducer hydrodynamic volume. Therefore, the dynamical magnetic signal of the transducer changes under an alternating magnetic field.

Further, it is also believed that when the analyte is multivalent, and said analyte interact with the recognition ligand of the functionalised magnetic nanoparticles, transducers (i.e. F-MNPs) agglomerate around the analyte. Such induced F-MNP agglomeration is responsible of the changes of dynamical magnetic signal under alternating magnetic field. In particular, it is believed that the agglomeration of the transducers around the analyte changes the magnetic relaxation mechanisms of the F-MNPs (both, Néel and Brownian processes) producing a variation in the dynamical magnetisation measurements with respect to the signal measured when the analyte is absent in the aqueous or biological fluid.

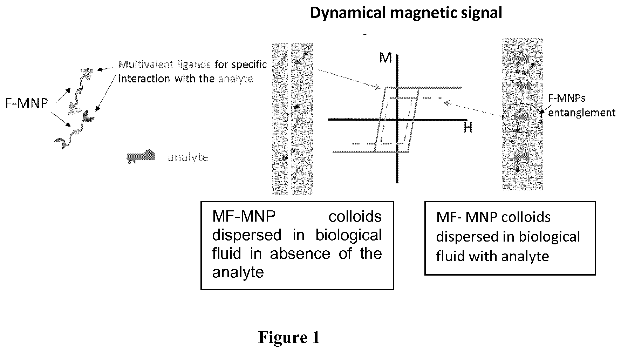

Figure 1 schematically compares the dynamical magnetisation measurements of F-MNPs dispersed in biological fluids containing a multivalent analyte with the corresponding measurement of the biological fluid when the analyte is not present. The figure represents the hysteresis loop variation when the analyte is present with respect to the signal measured for F-MNPs dispersed in the aqueous or biological fluid when the analyte is absent.

Such magnetisation changes of F-MNPs are accurately detected and quantified with a high sensitivity through the hysteresis loops: the area and/or the related magnetic parameters (i.e. remanence, coercitivity, maximal magnetisation).

Hence, the dynamical magnetisation signal of transducers dispersed in aqueous or biological fluids displays the interaction of F-MNPs with the analyte. The dynamical magnetic measurement may be performed by an apparatus comprising an AC magnetometer, preferably by an apparatus comprising a Faraday-Lenz inductive magnetometer under alternating magnetic fields (~100 kHz) in a few seconds after transducer incubation in the media where the analyte is present for 30-60 minutes at 37°C.

In a particular embodiment, the dynamical magnetization signal of the present invention is measured by an apparatus comprising an AC magnetometer; preferably by an apparatus comprising a Faraday-Lenz inductive magnetometer; even more preferably by using an an apparatus comprising an AC magnetometer for measuring the dynamical magnetisation signal of functionalized magnetic nanoparticles comprising:
a) an AC magnetic field generator configured to magnetically excite the functionalized magnetic nanoparticles, said AC magnetic field generator comprising a Litz wire coil as an excitation coil, wherein the AC magnetic field generator is part of a LCR circuit allowing to resonantly inject an AC current of a single resonant frequency to the Litz wire coil generating an AC magnetic field wherein the single resonant frequency is within the frequency range from 10 Hz to 1 MHz,
b) a magnetic flux detector comprising two counterwise wounded pick-up coils connected in series and mounted inside the excitation coil, wherein the two pick-up coils have the same turns and dimensions, and
c) a voltage reader,
wherein the voltage reader monitors the voltage signal of the pick-up coils of the magnetic flux detector.

The dynamical magnetisation signal of the method of the present invention, is measured as follows:
I. by measuring the induced voltage signals as a function of time under alternating magnetic fields for:
   - the functionalized magnetic nanoparticles in the aqueous or biological fluid of step b) containing the analyte under an alternating magnetic field; and/or for
   - the functionalized magnetic particle of step a) of a reference sample containing the aqueous or biological fluid without the analyte under an alternating magnetic field; and
II. by obtaining the hysteresis loop from said induced voltage signals as a function of time for
   - the functionalized magnetic nanoparticles in the aqueous or biological fluid of step b) containing the analyte under an alternating magnetic field; and/or for
   - the functionalized magnetic particle of step a) of a reference sample containing the aqueous or biological fluid without the analyte under an alternating magnetic field; and
the comparison of the dynamical magnetisation signal of step d) is performed by comparing the hysteresis loops; preferably by comparing the values of the hysteresis loop parameters; more preferably by comparing the areas of the hysteresis loops.

The dynamical magnetisation signal of the method of the present invention is measured under alternating magnetic fields at a single resonant frequency; preferably at a single resonant frequency and intensity.

In a preferred embodiment, the dynamical magnetisation signal of the method of the present invention, is measured as follows:
I. by measuring the induced voltage signals as a function of time under alternating magnetic fields for:
   - the functionalized magnetic nanoparticles in the aqueous or biological fluid of step b) containing the analyte under an alternating magnetic field; and/or for
   - the functionalized magnetic particle of step a) of a reference sample containing the aqueous or biological fluid without the analyte under an alternating magnetic field; and
II. by obtaining the hysteresis loop from said induced voltage signals as a function of time for
   - the functionalized magnetic nanoparticles in the aqueous or biological fluid of step b) containing the analyte under an alternating magnetic field; and/or for
   - the functionalized magnetic particle of step a) of a reference sample containing the aqueous or biological fluid without the analyte under an alternating magnetic field.

In a more preferred embodiment, the dynamical magnetisation signal of the aqueous or biological fluid measured in step c) is compared with a reference value to detect and/or quantify the presence of the analyte in the aqueous o biological fluid in step d) of the method of the present invention as follows:
by comparing the hysteresis loop of the aqueous or biological fluid measured in step c) with the hysteresis loop of the reference value; preferably by comparing the values of the hysteresis loop parameters obtained from said hysteresis loop.

In a particular embodiment, the hysteresis loop parameters of the method of the present invention are the magnetic hysteresis loop parameters; preferably are the area, remanence, coercitivity, maximal magnetisation, and/or magnetization harmonics.

In a more particular embodiment the hysteresis loop parameter of the method of the present invention is the hysteresis loop area.

In another particular embodiment the hysteresis loop parameter of the method of the present invention is the remanence.

In another particular embodiment the hysteresis loop parameter of the method of the present invention is the coercitivity.

In a more particular embodiment the hysteresis loop parameter of the method of the present invention is the maximal magnetisation.

In a more particular embodiment the hysteresis loop parameter of the method of the present invention is the magnetization harmonic.

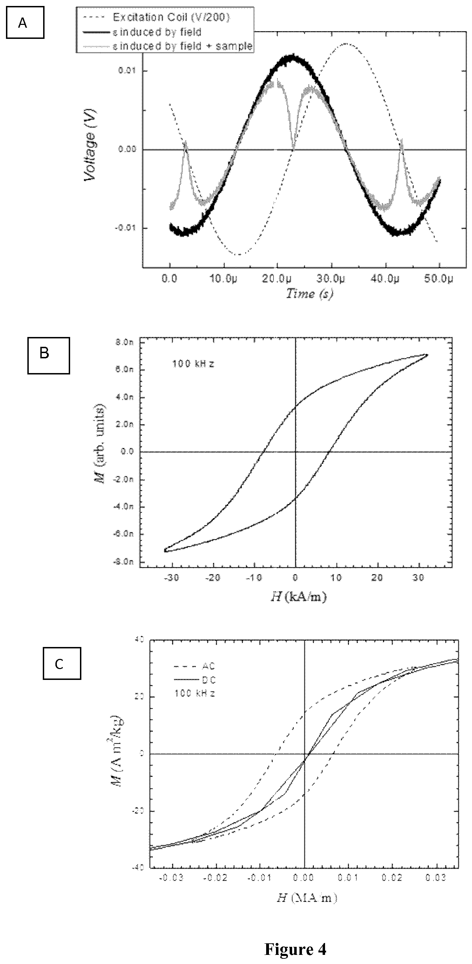

In the context of the present invention, the expression "induced voltage signals as a function of time" is related to the data directly measured under alternating magnetic fields with an apparatus comprising an AC magnetometer in the present invention (for example as in Figure 4a of the present invention).

In the context of the present invention the term "hysteresis loop" is related to a magnetic hysteresis loop (M) represented versus magnetic field intensity values as known in the art under an alternating magnetic field and preferably obtained from the induced voltage signals as a function of time measured with an apparatus comprising an AC magnetometer under alternating magnetic fields. The person skilled in the art knows how to obtain the hysteresis loop from the induced voltage signals as a function of time measured with an apparatus comprising an AC magnetometer under alternating magnetic fields.

The "hysteresis loop area" of the present invention may be obtained from the hysteresis loop by methods known in the art.

The term "remanence" in the context of the present invention refers to the remanent magnetization value when the external magnetic field is zero and is obtained from the hysteresis loop by methods known in the art.

The term "coercitivity" in the context of the present invention refers to the magnetic coercitivity, coercive field or coercive force as known in the art when the external magnetic field is zero and is obtained from the hysteresis loop by methods known in the art.

The expression "maximal magnetisation" in the context of the present invention refers to the magnetization value at the maximum external magnetic field applied and is obtained from the hysteresis loop by methods known in the art.

The expression "magnetization harmonics" in the context of the present invention are related to the nₜₕ order odd harmonic components of the magnetization with their corresponding magnitudes and phases as disclosed in [K. Murase, et al., Radiol. Phys. Technol. 6, 399 (2013)], which are obtained from the Fast Fourier Transform of the induced voltage signals as function of time by methods known in the art.

In a particular embodiment, the hysteresis loop of the present invention is a magnetic hysteresis loop (M) represented versus magnetic field intensity values; preferably is a magnetic hysteresis loop (M) represented versus magnetic field intensity values; wherein said magnetic field intensity values are from -300 kA/m to + 300 kA/m; preferably from -100 kA/m to + 100 kA/m; more preferably from -50 kA/m to + 50 kA/m; even more preferably from -40 kA/m to +40 kA/m.

It has been observed that changes of hysteresis loop caused by changes in the concentration of the analyte binding to the functionalized magnetic nanoparticles of the present invention, may be used to quantify the presence of an analyte in the aqueous o biological fluid, for example by comparing with a calibration curve.

In a particular embodiment, the method for *in vitro* detection and/or quantification of an analyte in aqueous or biological fluids of the present invention comprises:
a) providing functionalized magnetic nanoparticles, wherein each functionalized magnetic nanoparticle comprises a magnetic nanoparticle and a recognition ligand, wherein the magnetic nanoparticle has an average size of from 1 to 100 nm and a saturation magnetisation comprised between 20 and 300 emu/g, and wherein the recognition ligand is linked to said magnetic nanoparticle,
b) incubating the functionalized magnetic nanoparticles of step a) with an aqueous or biological fluid containing the analyte in conditions suitable for producing the binding of said functionalized magnetic nanoparticles to the analyte,
c) measuring the induced voltage signals as a function of time of the functionalized nanoparticles dispersed in an aqueous or biological fluid of step b) containing the analyte under an alternating magnetic field, and obtaining the hysteresis loop from said induced voltage signals as a function of time; and
d) comparing the hysteresis loop of the aqueous or biological fluid measured in step c) with a reference value to detect and/or quantify the presence of the analyte in the aqueous o biological fluid; and

wherein the reference value in step d) is the hysteresis loop obtained from the induced voltage signals as a function of time measured for the functionalized magnetic particle of step a) of a reference sample containing the aqueous or biological fluid without the analyte under an alternating magnetic field;
wherein the induced voltage signals as a function of time of steps c) and d) are measured with an apparatus comprising an AC magnetometer.

In a particular embodiment, the method for *in vitro* detection and/or quantification of an analyte in aqueous or biological fluids of the present invention comprises:
a) providing functionalized magnetic nanoparticles, wherein each functionalized magnetic nanoparticle comprises a magnetic nanoparticle and a recognition ligand, wherein the magnetic nanoparticle has an average size of from 1 to 100 nm and a saturation magnetisation comprised between 20 and 300 emu/g, and wherein the recognition ligand is linked to said magnetic nanoparticle,
b) incubating the functionalized magnetic nanoparticles of step a) with an aqueous or biological fluid containing the analyte in conditions suitable for producing the binding of said functionalized magnetic nanoparticles to the analyte,
c) measuring the induced voltage signals as a function of time of the functionalized nanoparticles dispersed in an aqueous or biological fluid of step b) containing the analyte under an alternating magnetic field, and obtaining the parameters of the hysteresis loop obtained from said induced voltage signals as a function of time; and
d) comparing the values of the parameters of the hysteresis loop of the aqueous or biological fluid measured in step c) with a reference value to detect and/or quantify the presence of the analyte in the aqueous o biological fluid; and

wherein the reference value in step d) is the values of the parameters of the hysteresis loop obtained from the hysteresis loop obtained from the induced voltage signals as a function of time measured for the functionalized magnetic particle of step a) of a reference sample containing the aqueous or biological fluid without the analyte under an alternating magnetic field;
wherein the induced voltage signals as a function of time of steps c) and d) are measured with an apparatus comprising an AC magnetometer.

In a more particular embodiment, the method for *in vitro* detection and/or quantification of an analyte in aqueous or biological fluids of the present invention comprises:
a) providing functionalized magnetic nanoparticles, wherein each functionalized magnetic nanoparticle comprises a magnetic nanoparticle and a recognition ligand, wherein the magnetic nanoparticle has an average size of from 1 to 100 nm and a saturation magnetisation comprised between 20 and 300 emu/g, and wherein the recognition ligand is linked to said magnetic nanoparticle,
b) incubating the functionalized magnetic nanoparticles of step a) with an aqueous or biological fluid containing the analyte in conditions suitable for producing the binding of said functionalized magnetic nanoparticles to the analyte,
c) measuring the induced voltage signals as a function of time of the functionalized nanoparticles dispersed in an aqueous or biological fluid of step b) containing the analyte under an alternating magnetic field, and obtaining the hysteresis loop area from the hysteresis loop obtained from the induced voltage signals as a function of time; and
d) comparing the hysteresis loop area of the aqueous or biological fluid measured in step c) with a reference value to detect and/or quantify the presence of the analyte in the aqueous o biological fluid; and

wherein the reference value in step d) is the hysteresis loop area obtained from the hysteresis loop obtained from the induced voltage signals as a function of time measured for the functionalized magnetic particle of step a) of a reference sample containing the aqueous or biological fluid without the analyte under an alternating magnetic field;
wherein the induced voltage signals as a function of time of steps c) and d) are measured with an apparatus comprising an AC magnetometer.

In another more particular embodiment, the method for *in vitro* detection and/or quantification of an analyte in aqueous or biological fluids of the present invention comprises:
a) providing functionalized magnetic nanoparticles, wherein each functionalized magnetic nanoparticle comprises a magnetic nanoparticle and a recognition ligand, wherein the magnetic nanoparticle has an average size of from 1 to 100 nm and a saturation magnetisation comprised between 20 and 300 emu/g, and wherein the recognition ligand is linked to said magnetic nanoparticle,
b) incubating the functionalized magnetic nanoparticles of step a) with an aqueous or biological fluid containing the analyte in conditions suitable for producing the binding of said functionalized magnetic nanoparticles to the analyte,
c) measuring the induced voltage signals as a function of time of the functionalized nanoparticles dispersed in an aqueous or biological fluid of step b) containing the analyte under an alternating magnetic field, and obtaining the magnetization harmonics obtained from the induced voltage signals as a function of time; and
d) comparing the magnetization harmonics of the aqueous or biological fluid measured in step c) with a reference value to detect and/or quantify the presence of the analyte in the aqueous o biological fluid; and

wherein the reference value in step d) is the magnetization harmonics obtained from the the induced voltage signals as a function of time measured for the functionalized magnetic particle of step a) of a reference sample containing the aqueous or biological fluid without the analyte under an alternating magnetic field;
wherein the induced voltage signals as a function of time of steps c) and d) are measured with an apparatus comprising an AC magnetometer.

The term "display" refers to visualise the changes of the physical parameter varying after the specific interaction with the analyte enabling to show the detection. In the present invention, the term "display" refers to quantify the variation of the dynamical magnetisation signals of F-MNP colloids dispersed in aqueous or biological fluids in presence/absence of the analyte under alternating magnetic fields.

In one particular embodiment, in step c) of the method of the invention the dynamical magnetisation signal of the functionalised magnetic nanoparticles in the aqueous or biological fluid of step b) is measured by an AC magnetometer or a Faraday-Lenz inductive magnetometer. The Faraday-Lenz inductive magnetometer accurately displays the specific interaction between ligands and analyte. Without being bound to any theory it is believed that the dynamical magnetisation signal detected on F-MNPs under alternating magnetic fields (~100 kHz) is proportional to the imaginary part of the magnetic susceptibility. Said AC magnetic susceptibility is tightly related to relaxation processes of F-MNPs which are extremely sensitive to the interaction between F-MNPs and analyte. The F-MNPs entanglement when the analyte is multivalent favours magnetic dipolar interactions, which consequently influence their relaxation mechanisms. Both phenomena lead to variation of the dynamical magnetic response, which allow accurately monitoring and quantifying the detection of analytes.

In step d) of the method of the invention the dynamical magnetisation signal of the aqueous or biological fluid containing the analyte measured in step c) is compared with a reference value to detect and/or quantify the presence of the analyte in the aqueous o biological fluid. In particular, the detection of an analyte in the aqueous or biological fluid by the method of the invention may be indicative of a certain disease.

In a particular embodiment, the reference value in step d) is that resulting from measuring the dynamical magnetisation signals of the functionalized magnetic particle of step a) of a reference sample containing the F-MNPs dispersed in the aqueous or biological fluid without the analyte under alternating magnetic fields. In another particular embodiment, the reference value in step d) is that resulting from measuring the dynamical magnetisation signals of the functionalized magnetic particle of step a) of a reference sample containing the F-MNPs dispersed in the aqueous or biological fluid containing a known concentration of the analyte under alternating magnetic fields.

One aspect of the invention refers to the use of functionalized magnetic nanoparticles for *in vitro* detection and/or quantification of an analyte in aqueous or biological fluids as defined in claim 8, wherein each functionalized magnetic nanoparticle comprises a magnetic nanoparticle and a recognition ligand, wherein the magnetic nanoparticle has an average size of from 1 to 100 nm and a saturation magnetisation comprised between 20 and 300 emu/g, wherein the recognition ligand is linked to said magnetic nanoparticle, and wherein said analyte is detected in aqueous or biological fluids according to the method for *in vitro* detection and/or quantification of an analyte in aqueous or biological fluids above disclosed comprising:
a) providing functionalized magnetic nanoparticles, wherein each functionalized magnetic nanoparticle comprises a magnetic nanoparticle and a recognition ligand, wherein the magnetic nanoparticle has an average size of from 1 to 100 nm and a saturation magnetisation comprised between 20 and 300 emu/g, and wherein the recognition ligand is linked to said magnetic nanoparticle,
b) incubating the functionalized magnetic nanoparticles of step a) with an aqueous or biological fluid containing the analyte in conditions suitable for producing the binding of said functionalized magnetic nanoparticles to the analyte,
c) measuring the dynamical magnetisation signal of the functionalized magnetic nanoparticles in the aqueous or biological fluid of step b) containing the analyte under an alternating magnetic field, and
d) comparing the dynamical magnetisation signal of the aqueous or biological fluid measured in step c) with a reference value to detect and/or quantify the presence of the analyte in the aqueous o biological fluid; and wherein the reference value in step d) is that resulting from measuring the dynamical magnetisation signal of the functionalized magnetic nanoparticle of step a) of a reference sample containing the aqueous or biological fluid without the analyte under an alternating magnetic field; and
wherein the dynamical magnetisation signal of steps c) and d) are measured with an apparatus comprising an AC magnetometer.

In a particular embodiment the functionalized magnetic nanoparticle for *in vitro* detection and/or quantification of an analyte in aqueous or biological fluids, is an iron oxide nanoparticle functionalised with a peptide.

When the analyte to be detected and/or quantified is monovalent, in step b) analyte interacts with the recognition ligand of the functionalised magnetic nanoparticles, the Brownian relaxation mechanism is altered, as consequence of the increase in the hydrodynamic volume after the specific binding to the analyte. Therefore, the dynamical magnetic signal under an alternating magnetic field changes, for example with respect to the reference value of a sample obtained in absence of the analyte.

Further, as Figure 1 shows, when multivalent analyte and the recognition ligand of the functionalised magnetic nanoparticle interact in step b), transducers (i.e.F-MNPs) do agglomerate around the analyte favouring the entanglement of the nanoparticles around the multivalent analyte. The F-MNP agglomeration around the multivalent analyte influences the magnetic moment relaxation of the F-MNP assembly resulting in a variation of the dynamical magnetisation measurements under alternating magnetic fields.

Such changes of the AC magnetisation are accurately detected and quantified through the hysteresis loops: the area and/or the related magnetic parameters (i.e. remanence, coercitivity, maximal magnetisation). Hence, the dynamical magnetisation signal of transducers dispersed in aqueous or biological fluids displays the interaction of F-MNPs with the analyte.

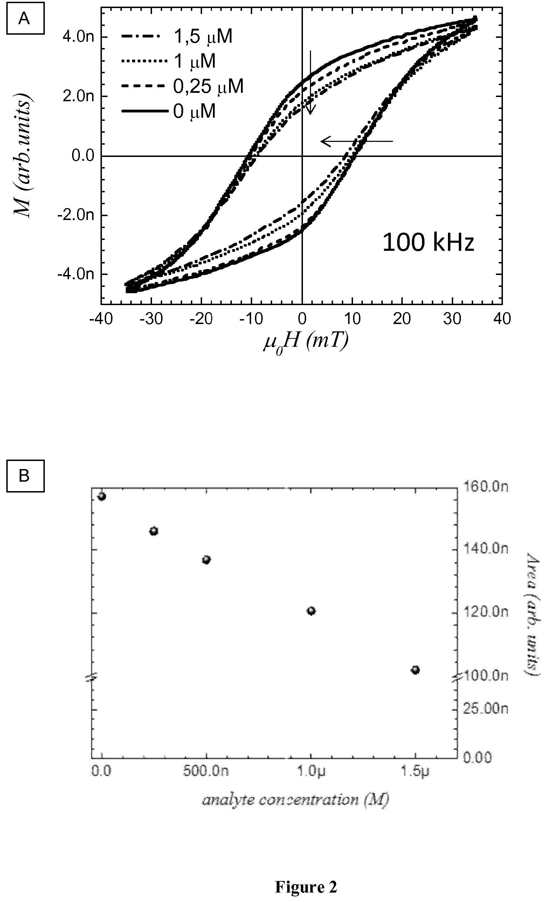

In order to quantify the amount of analyte present in the sample, calibration curves at different concentrations may be prepared from the dynamical magnetisation signal of the original biological fluid containing different known analyte concentrations. The calibration curves may be compared with the signal of the sample to be tested. Thus, for example the corresponding transducer (i.e. F-MNPs) may be dispersed in the biological fluids at concentrations between 0.5-5 g/L. Then, calibration curves may be plotted from the dynamical magnetisation data measured at different concentrations. Said calibration curves can be used for quantifying the amount of analyte by comparing with the dynamical magnetisation curve obtained in the real sample. Figure 2A shows the analyte concentration dependence of the dynamical magnetisation measurements for F-MNP dispersed in phosphate buffer saline at 1 mg Fe/mL, 100 kHz and 35 mT. Figure 2B shows the analyte concentration dependence of the area extracted from figure 2A. The results of the figure are obtained from a transducer based on a dimercaptosuccinic acid coated 12 nm iron oxide nanoparticles functionalized with the GST-MEEVF peptide (acting as recognition molecule) that can specifically bind dimeric VFP_{d}-TPR protein (acting as analyte).

### Measuring the efficacy of a treatment of a disease

One aspect of the invention refers to a method for measuring the efficacy of a treatment of a disease in a subject, comprising:
a) providing functionalized magnetic nanoparticles, wherein each functionalized magnetic nanoparticle comprises a magnetic nanoparticle and a recognition ligand, wherein the magnetic nanoparticle has an average size of from 1 to 100 nm and a saturation magnetisation comprised between 20 and 300 emu/g, and wherein the recognition ligand is linked to said magnetic nanoparticle,
b) incubating the functionalized magnetic nanoparticles of step a) with an aqueous or biological fluid containing an analyte from the subject in conditions suitable for producing the binding of said functionalized magnetic nanoparticles to said analyte,
c) measuring the dynamical magnetisation signal of the functionalized magnetic nanoparticles in the aqueous or biological fluid from the subject of step b) under an alternating magnetic field, and measuring the dynamical magnetisation signal of a reference sample under an alternating magnetic field, wherein said reference sample is obtained from the same subject at an earlier time of point of the disease or prior to the disease, and
d) comparing the dynamical magnetisation signal of the aqueous or biological fluid of the treated subject and that of the reference sample measured in step c), wherein a change of the dynamical magnetic signal of the treated subject with respect to the dynamical magnetic signal of the reference sample is indicative of the efficacy of a treatment of a disease in a subject; and

wherein the dynamical magnetisation signals of step c) are measured with an apparatus comprising an AC magnetometer; and
wherein the dynamical magnetisation signal is measured as follows:
   I. by measuring the induced voltage signals as a function of time under alternating magnetic fields for:
      - the functionalized magnetic nanoparticles in the aqueous or biological fluid of step b) containing the analyte under an alternating magnetic field; and/or for
      - the functionalized magnetic nanoparticle of step a) of a reference sample containing the aqueous or biological fluid without the analyte under an alternating magnetic field; and
   II. by obtaining the hysteresis loop from said induced voltage signals as a function of time for
      - the functionalized magnetic nanoparticles in the aqueous or biological fluid of step b) containing the analyte under an alternating magnetic field; and/or for
      - the functionalized magnetic nanoparticle of step a) of a reference sample containing the aqueous or biological fluid without the analyte under an alternating magnetic field;
      wherein the comparison of the dynamical magnetisation signal of step d) is performed by comparing the hysteresis loops; preferably by comparing the values of the hysteresis loop parameters obtained from the hysteresis loops; and
wherein the dynamical magnetisation signal is measured under alternating magnetic fields at a single resonant frequency.

**In** a particular embodiment, the magnetic nanoparticle provided in step a) is activated for the immobilisation of a recognition ligand. In preferred embodiment, the magnetic nanoparticle is coated with an organic hydrophilic compound to be activated for the immobilisation of a targeting agent. The organic hydrophilic coating on the magnetic nanoparticle minimizes the non-specific adsorption of molecules from complex biological fluids maximizing the sensitivity of the detection method. Further, the organic hydrophilic coating of the magnetic nanoparticle preserves the colloidal stability in biological fluids (such as blood, plasma, saliva, urine, etc) preventing the non-specific adsorption of biological molecules such as proteins, so that the functionalised magnetic nanoparticle is stable in aqueous and biological fluids without significant variation of their hydrodynamic size (< 10% variation). Examples of hydrophilic coatings include polyethylene glycol, carboxylated polyethylene glycol, amidated polyethylene glycol, chitosan, oligosaccharide-based coatings, and serum albumin protein among others. Furthermore, the functionalization of the magnetic nanoparticles with recognition ligands (i.e. receptor) does not affect the colloidal stability of the magnetic nanoparticles nor the recognition properties of the "analyte" in the biological fluids. Suitable hydrophilic coatings for the magnetic nanoparticle contain carboxylic acids, PMAO, amine groups, alcohol groups, thiol groups, maleimide, etc. The preservation of a neutral net surface charge of the F-MNPs also provides the best features to minimize the unspecific interactions with biological molecules, and consequently preserving their colloidal stability in biological fluids.

According to step b) the functionalized magnetic nanoparticles of step a) are incubated with an aqueous or biological fluid from the subject containing an analyte in conditions suitable for producing the binding of said functionalized magnetic nanoparticles to said analyte.

The term "analyte", as used herein, refers to any biomarker molecule without limitation. Analytes or biomarkers suitable for the method of the invention include any macromolecules or small molecule. In a particular embodiment the analyte is selected from drugs, doping agents, proteins, peptides, nucleic acids, nucleic acid-protein complexes, mRNA, microRNA, lipids, vesicles, vesicle markers, or cancerous cell. In a particular embodiment the analyte is selected from proteins, peptides, pseudopeptides, amino acids, amino acids derivatives, nucleic acids, nucleic acid-protein complexes, mRNA, microRNA, sugars, lipids, vesicles, vesicle markers, alkaloids, glycosides, lipids, non-ribosomal peptides, phenazines, natural phenols, polyketide, terpenes, and tetrapyrroles. In a particular embodiment the analyte is a drug. In a preferred embodiment, the biological fluid from the subject is blood, and the analyte contained in the biological fluid is a drug.

In step c), the dynamical magnetisation signal of the functionalized magnetic nanoparticles is measured in the aqueous or biological fluid from the subject of step b) under an alternating magnetic field, and the dynamical magnetisation signal of a reference sample is measured under an alternating magnetic field, wherein said reference sample is obtained from the same subject at an earlier time of point of the disease or prior to the disease.

In one particular embodiment, in step c) of the method of the invention the dynamical magnetisation signal of the functionalised magnetic nanoparticles in the aqueous or biological fluid from the subject of step b), and the dynamical magnetisation signal of a reference sample is measured under an alternating magnetic field by an AC magnetometer or a Faraday-Lenz inductive magnetometer. The Faraday-Lenz inductive magnetometer accurately displays the specific interaction between ligands and analyte.

According to step d) the dynamical magnetisation signal of the aqueous or biological fluid of the treated subject and that of the reference sample measured in step c) are compared, so that a change of the dynamical magnetic signal of the aqueous or biological fluid of the treated subject with respect to the dynamical magnetic signal of the reference sample is indicative of the efficacy of a treatment of a disease in a subject.

The term "changes of the dynamical magnetic signal" refers to changes of the hysteresis loops acquired when F-MNPs are subjected to alternating magnetic fields. Those changes in the hysteresis loops are related to the area, and/or the related magnetic parameters (i.e. remanence, coercitivity, maximal magnetisation). Such magnetisation changes are accurately detected and quantified with a high sensitivity through the hysteresis loops: the area and/or the related magnetic parameters (i.e. remanence, coercitivity, maximal magnetisation).

Thus, in a particular embodiment a change in the hysteresis loops is detected when the dynamical magnetic signal of the aqueous or biological fluid of the treated subject is compared with the dynamical magnetic signal of the reference sample. A change in the dynamical signal is indicative of the efficacy of a treatment of a disease in a subject. In particular, a change in the dynamical signal is related to the changes in the levels of a biomarker related to a specific disease.

In another particular embodiment a change in the hysteresis loops is not detected when the dynamical magnetic signal of the aqueous or biological fluid of the treated subject is compared with the dynamical magnetic signal of the reference sample. When the dynamical magnetic signal of the aqueous or biological fluid of the treated subject with respect to the dynamical magnetic signal of the reference sample remains constant, constant levels of a biomarker related to a specific disease are expected. Thus, for example, when the biological fluid from the treated subject is blood containing a drug as analyte, a constant dynamical magnetic signal is indicative of constant levels of a drug in the blood.

The dynamical magnetic signal of the method for measuring the efficacy of a treatment of a disease in a subject of the present invention is measured as described for the method for *in vitro* detection and/or quantification of an analyte in aqueous or biological fluids of the present invention in any of its particular embodiments.

In a particular embodiment, the dynamical magnetisation signal of the method for measuring the efficacy of a treatment of a disease in a subject of the present invention, is measured as follows:
I. by measuring the induced voltage signals as a function of time under alternating magnetic fields for:
   - the dynamical magnetisation signal of the functionalized magnetic nanoparticles in the aqueous or biological fluid from the subject of step b) under an alternating magnetic field; and/or for
   - a reference sample under an alternating magnetic field; and
II. by obtaining the hysteresis loop from said induced voltage signals as a function of time for
   - the dynamical magnetisation signal of the functionalized magnetic nanoparticles in the aqueous or biological fluid from the subject of step b) under an alternating magnetic field; and/or for
   - a reference sample under an alternating magnetic field; and
the comparison of the dynamical magnetisation signal of step d) is performed by comparing the hysteresis loops; preferably by comparing the values of the hysteresis loop parameters; more preferably by comparing the areas of the hysteresis loops.

The hysteresis loop parameters of the method for measuring the efficacy of a treatment of a disease in a subject of the present invention, are those described for the method for *in vitro* detection and/or quantification of an analyte in aqueous or biological fluids of the present invention in any of its particular embodiments.

In a particular embodiment, the dynamical magnetization signal of the method for measuring the efficacy of a treatment of a disease in a subject of the present invention, is measured by an apparatus comprising an AC magnetometer; preferably by an apparatus comprising a Faraday-Lenz inductive magnetometer; even more preferably by using an an apparatus comprising an AC magnetometer for measuring the dynamical magnetisation signal of functionalized magnetic nanoparticles comprising:
a) an AC magnetic field generator configured to magnetically excite the functionalized magnetic nanoparticles, said AC magnetic field generator comprising a Litz wire coil as an excitation coil, wherein the AC magnetic field generator is part of a LCR circuit allowing to resonantly inject an AC current of a single resonant frequency to the Litz wire coil generating an AC magnetic field wherein the single resonant frequency is within the frequency range from 10 Hz to 1 MHz,
b) a magnetic flux detector comprising two counterwise wounded pick-up coils connected in series and mounted inside the excitation coil, wherein the two pick-up coils have the same turns and dimensions, and
c) a voltage reader,
wherein the voltage reader monitors the voltage signal of the pick-up coils of the magnetic flux detector.

In a more particular embodiment, the dynamical magnetization signal of the method for measuring the efficacy of a treatment of a disease in a subject of the present invention is measured with the apparatus of the present invention in any of its particular embodiments.

The method for measuring the efficacy of a treatment of a disease in a subject provided in the present application allows administering efficient therapeutic doses to the treated subject. Further, the functionalized magnetic nanoparticles in the method for measuring the efficacy of a treatment of a disease in a subject provided in the present application, can act as an innovative sensor for detecting an analyte in an aqueous o biological fluid without requiring additional steps for preparing the sample. In one particular embodiment, the functionalised magnetic particle can be used for monitoring the variation of the dynamical magnetic response of F-MNPs into blood, urine, cells or tissues, thus, providing a new way of quantifying the variation of dynamical magnetic features of given MNPs into any biological matrices or fluids.

Another aspect of the invention also relates to the use of functionalized magnetic nanoparticles for measuring the efficacy of a treatment of a disease in a subject, wherein each functionalized magnetic nanoparticle comprises a magnetic nanoparticle and a recognition ligand, wherein the magnetic nanoparticle has an average size of from 1 to 100 nm and a saturation magnetisation comprised between 20 and 300 emu/g, wherein the recognition ligand is linked to said magnetic nanoparticle, and wherein the efficacy of said treatment is measured according to the method for measuring the efficacy of a treatment of a disease in a subject above disclosed. Said functionalized magnetic nanoparticles can be used for measuring the efficacy of any disease in which the prognosis is related to the level of one or several biomarker in biological fluids. In a particular embodiment, the invention relates to the use of said functionalized magnetic nanoparticles disclosed for measuring the efficacy of a treatment of a disease in a subject, wherein the disease is selected from cancer, autoimmune diseases, neurodegenerative diseases, cardiovascular diseases, inflammatory diseases, and endocrine diseases.

### Diagnosis of a disease

In another aspect, the invention relates to the *in vitro* use of functionalized magnetic nanoparticles for diagnosing a disease, wherein each functionalised magnetic nanoparticle comprises a magnetic nanoparticle and a recognition ligand, wherein the magnetic nanoparticle has an average size of from about 1 to about 100 nm and a saturation magnetisation comprised between 20 and 300 emu/g, wherein the recognition ligand is linked to said magnetic nanoparticle, and wherein the disease is diagnosed by *in vitro* detection and/or quantification of an analyte in aqueous or biological fluids according to the method of the invention above disclosed. In a particular embodiment, the disease to be diagnosed is selected from any disease whose diagnosis is related to the level of one or several biomarker in biological fluids, including cancer, autoimmune diseases, neurodegenerative diseases, cardiovascular diseases, inflammatory diseases, and endocrine diseases, among others.

The proposed methodology may be used to *in vitro* detect and quantify the amount of analyte or biomarker present in aqueous or biological fluids according to the method of the invention above disclosed. In particular, the functionalized magnetic nanoparticle can be advantageously used for *in vitro* detection of the presence of the analyte or biomarker in *ex vivo* samples of small volume of aqueous or biological fluids, according to the method of the invention above disclosed.

Moreover, the dynamical magnetic signal of said functionalized magnetic nanoparticles can also be used for *in vitro* detection of unspecific interactions between the transducer and biomolecules present in a dispersion media according to the method of the invention above disclosed, opening a new manner to engineer the MNP surface for inhibiting unspecific interaction between MNP surface and given molecules.

Another aspect of the invention refers to a method of diagnosis of a disease in a subject comprising the following steps:
a) providing functionalized magnetic nanoparticles, wherein each functionalized magnetic nanoparticle comprises a magnetic nanoparticle and a recognition ligand, wherein the magnetic nanoparticle has an average size of from 1 to 100 nm and a saturation magnetisation comprised between 20 and 300 emu/g, and wherein the recognition ligand is linked to said magnetic nanoparticle,
b) incubating the functionalized magnetic nanoparticles of step a) with an aqueous or biological fluid from the subject in conditions suitable for producing the binding of said functionalized magnetic nanoparticles to an analyte, wherein said analyte is a biomarker of the disease to be diagnosed,
c) measuring the dynamical magnetisation signal of the functionalized magnetic nanoparticles in the aqueous or biological fluid from the subject of step b) under an alternating magnetic field, and
d) comparing the dynamical magnetisation signal of the aqueous or biological fluid of the subject measured in step c) with a reference value indicative of the disease to be diagnosed; and
wherein the dynamical magnetisation signals are measured with an apparatus comprising an AC magnetometer; and
wherein the dynamical magnetisation signal is measured as follows:
I. by measuring the induced voltage signals as a function of time under alternating magnetic fields for:
   - the functionalized magnetic nanoparticles in the aqueous or biological fluid of step b) containing the analyte under an alternating magnetic field; and/or for
   - the functionalized magnetic nanoparticle of step a) of a reference sample containing the aqueous or biological fluid without the analyte under an alternating magnetic field; and
II. by obtaining the hysteresis loop from said induced voltage signals as a function of time for
   - the functionalized magnetic nanoparticles in the aqueous or biological fluid of step b) containing the analyte under an alternating magnetic field; and/or for
   - the functionalized magnetic nanoparticle of step a) of a reference sample containing the aqueous or biological fluid without the analyte under an alternating magnetic field; and

   wherein the comparison of the dynamical magnetisation signal of step d) is performed by comparing the hysteresis loops; preferably by comparing the values of the hysteresis loop parameters obtained from the hysteresis loops; and
   wherein the dynamical magnetisation signal is measured under alternating magnetic fields at a single resonant frequency.

In a particular embodiment, the reference value in step d) is that resulting from measuring the dynamical magnetisation signals of the functionalized magnetic particle of step a) under alternating magnetic fields in a reference sample containing the F-MNPs dispersed in the aqueous or biological fluid containing an analyte, wherein the analyte is a biomarker corresponding to the disease to be diagnosed. In a preferred embodiment the biomarker is selected from drugs, proteins, peptides, nucleic acids, nucleic acid-protein complexes, mRNA, microRNA, lipids, vesicles, vesicle markers, or cancerous cell. In a more preferred embodiment the analyte or biomarker is selected from proteins, peptides, pseudopeptides, amino acids, amino acids derivatives, nucleic acids, nucleic acid-protein complexes, mRNA, microRNA, sugars, lipids, vesicles, vesicle markers, alkaloids, glycosides, lipids, non-ribosomal peptides, phenazines, natural phenols, polyketide, terpenes, and tetrapyrroles.

The dynamical magnetic signal of the method of diagnosis of a disease in a subject of the present invention is measured as described for the method for *in vitro* detection and/or quantification of an analyte in aqueous or biological fluids of the present invention in any of its particular embodiments.

In a particular embodiment, the dynamical magnetisation signal of the method of diagnosis of a disease in a subject of the present invention, is measured as follows:
I. by measuring the induced voltage signals as a function of time under alternating magnetic fields for:
   - the aqueous or biological fluid from the subject of step b) under an alternating magnetic field; and/or for
   - the reference value indicative of the disease to be diagnosed; and
II. by obtaining the hysteresis loop from said induced voltage signals as a function of time for
   - the aqueous or biological fluid from the subject of step b) under an alternating magnetic field; and/or for
   - the reference value indicative of the disease to be diagnosed; and
the comparison of the dynamical magnetisation signal of step d) is performed by comparing the hysteresis loops; preferably by comparing the values of the hysteresis loop parameters; more preferably by comparing the areas of the hysteresis loops.

The hysteresis loop parameters of method of diagnosis of a disease in a subject of the present invention are those described for the method for *in vitro* detection and/or quantification of an analyte in aqueous or biological fluids of the present invention in any of its particular embodiments.

In a particular embodiment, the dynamical magnetization signal of the method of diagnosis of a disease in a subject of the present invention is measured by an apparatus comprising an AC magnetometer; preferably by an apparatus comprising a Faraday-Lenz inductive magnetometer; even more preferably by using an an apparatus comprising an AC magnetometer for measuring the dynamical magnetisation signal of functionalized magnetic nanoparticles comprising:
a) an AC magnetic field generator configured to magnetically excite the functionalized magnetic nanoparticles, said AC magnetic field generator comprising a Litz wire coil as an excitation coil, wherein the AC magnetic field generator is part of a LCR circuit allowing to resonantly inject an AC current of a single resonant frequency to the Litz wire coil generating an AC magnetic field wherein the single resonant frequency is within the frequency range from 10 Hz to 1 MHz,
b) a magnetic flux detector comprising two counterwise wounded pick-up coils connected in series and mounted inside the excitation coil, wherein the two pick-up coils have the same turns and dimensions, and
c) a voltage reader,
wherein the voltage reader monitors the voltage signal of the pick-up coils of the magnetic flux detector.

In a more particular embodiment, the dynamical magnetization signal of the method of diagnosis of a disease in a subject of the present invention is measured with the apparatus of the present invention in any of its particular embodiments.

The method of diagnosis of a disease of the present invention can be used for diagnosing a disease selected from cancer, autoimmune diseases, neurodegenerative diseases, cardiovascular diseases, inflammatory diseases, and endocrine diseases, among others.

Biomarkers suitable for diagnosing a certain disease are well known in the art. Thus, for example L. A. Soares Nunes et al [Biochemia Medica 2015; 25(2):177-92] discloses some biomarkers present in saliva that are indicative of certain conditions.

The following table collects some exemplary biomarkers suitable for diagnosing certain diseases.

| **Biomarkers** | **Condition** |
|---|---|
| Urinary biomarkers: Albumin, β2-Microglobulin, Clusterin, Cystatin C, KIM-1, Total Protein, and Trefoil factor-3 | Drug-induced Nephrotoxicity |
| Urinary biomarkers: Clusterin, Renal Papillary Antigen (RPA-1) | Drug-induced Nephrotoxicity |
| Serum/plasma biomarkers: Cardiac troponins T (cTnT) and I (cTnI) | Drug-induced Cardiotoxicity |
| Serum/bronchoalveolar lavage fluid biomarker: Galactomannan | Invasive Aspergillosis |
| Plasma biomarker: Fibrinogen | Chronic Obstruction Pulmonary Disease (COPD) |

### Apparatus

One aspect of the invention relates to the apparatus designed for carrying out the methods of the invention for *in vitro* detection and/or quantification of an analyte in aqueous or biological fluids or for measuring the efficacy of a treatment of a disease in a subject comprising an AC magnetometer for measuring the dynamical magnetisation signal of functionalized magnetic nanoparticles comprising:
a) an AC magnetic field generator configured to magnetically excite the functionalized magnetic nanoparticles, said AC magnetic field generator comprising a Litz wire coil as an excitation coil, wherein the AC magnetic field generator is part of a LCR circuit allowing to resonantly inject an AC current of a single resonant frequency to the Litz wire coil generating an AC magnetic field wherein the single resonant frequency is within the frequency range from 10 Hz to 1 MHz,
b) a magnetic flux detector comprising two counterwise wounded pick-up coils connected in series and mounted inside the excitation coil, wherein the two pick-up coils have the same turns and dimensions, and
c) a voltage reader,

wherein the voltage reader monitors the voltage signal of the pick-up coils of the magnetic flux detector; and
wherein the excitation coil is immersed into a dielectric liquid flow that circulates across a heat exchanger for thermalization at room temperature.

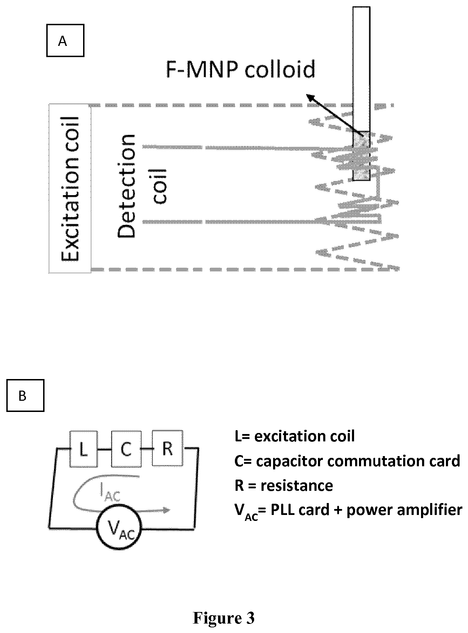

Figure 3A shows a schematic representation of the different parts of the AC magnetometer provided by the present invention. The AC magnetic field generator comprises a Litz wire coil as an excitation coil. An alternating (AC) sinusoidal current is injected through the excitation coil for generating a homogenous alternating magnetic field in a sample. Preferably, the sample presents a volume up to 4000 microlitres; preferably up to 400 microlitres. The excitation coil is the inductor (L) part of a LCR circuit (Figure 3B) where an AC current is resonantly injected at a single resonant frequency (*f*_{R}) given by the expression *f*_{R} = (LC)^{-1/2}/2π for reaching the suitable magnetic field intensity. Thus, the excitation coil (L) may be connected in serie to a capacitor (C) whose values range from 0.1 nF up to 20 mF, and to a resistance (R) ranging from 0.1 to 100 ohms. Thus, an AC current of a given resonant frequency value, preferably ranging from 300 mHz to 1MHz may be injected in the LCR circuit as shows figure 3B, preferably from 1 kHz to 0.5 MHz, more preferably between 5 kHz to 0.25 MHz.

The magnetic flux detector, also known as magnetic induction detector of the apparatus of the invention comprises two counterwise-wounded pick-up coils connected in series and mounted inside the excitation coil, wherein the two pick-up coils have the same turns and dimensions (Figure 3A). The term "detection coil" in the context of the invention refers to the circuit of two counterwise-wounded pick-up coils connected in series and mounted inside an excitation coil. Thus, the magnetic flux detector of the apparatus of the invention comprises a detection coil that probes the induced voltage signals as a function of time (Figure 4a). Such induced voltage signals as a function of time may be calibrated in magnetic units by comparing magnetisation values at given field intensity obtained under AC and quasi-static conditions (see Figure 4.c) as known in the art.

The counterwise-wounded pick-up coils are preferably made of a single diameter copper wire. Moreover, each pick-up coil has preferably a diameter ranging from 1 to 20 mm, and a height ranging from 0.5 to 20 mm. Both pick-up coils present the same turns and dimensions in order to compensate the electromotive force (EMF) induced inside the pick-up coils.

For the purpose of switching the resonant frequency of the alternating magnetic field, the inventors have found that a capacitor commutation card with up to 40 different capacitors allows automatizing the frequency commutation with a personal computer. In addition, the alternating current (I_{AC}) may be generated by a *phase-locked loop* (PLL) card and can be programmed with a personal computer. I_{AC} is previously amplified to be injected into the LCR circuit to achieve AC current values up to 150 A (peak values) leading to magnetic field intensities up to 45 kA/m. The excitation coil is efficiently cooled by immersing the coil into a dielectric liquid flow that circulates across a heat exchanger for thermalization at room temperature. The excitation coil of the apparatus of the present invention is immersed into a dielectric liquid flow that circulates across a heat exchanger for thermalization at room temperature.

In a particular embodiment, the apparatus of the present invention works at room temperature; preferably between 15 and 40 degrees Celsius.

The coils are connected to an acquisition system capable of digitalizing and monitoring the voltage signal. In particular, the detection coil is connected to a voltage reader system capable of digitalizing, and monitoring the induced voltage with (*Sₛₐₘₚ*) and without (*S_{back}*) the F-MNP colloid sample, into the upper pick-up coil. Immediately after, both acquired signals (*Sₛₐₘₚ* and *S_{back}*) are automatically analysed, quantified and plotted.

In a particular embodiment the sample may be firstly loaded in an automatized multiple sample holder. Said automatized multiple sample holder may have up to 20 different sample positions. When the sample is selected, the user can programme different field conditions (i.e. frequency and/or intensity) for the excitation coil during the AC magnetometry measurement (field condition mode). Also, multiple repetitions of AC magnetometry measurements on the same sample and field condition can be programmed (*repetition* mode). Once the *field condition* or *repetition* measurement runs, the excitation coil generates the AC magnetic according to the previously introduced specifications. This magnetic field is permanently monitored in order to ensure that it maintains stable as long as the measurement is carried out. Under these conditions, accordingly to the Faraday's law of induction, an electromotive force (EMF) is induced inside the pick-up, since a variable-in-time magnetic field goes through them. As the pick-up coils are not fully compensated, a residual background voltage signal (*S_{back}*) is obtained, and monitored (i.e. acquiring and digitalizing the EMF with both empty pick-up coils) as shown in Figure 4A. Just after, the automatized sample holder places the colloidal dispersed F-MNP sample inside the upper pick-up coil, producing a change in the induced signal "*S_{back+samp}*"*.* Simultaneously, the software operates with both voltage signals as follows: *S_{back}* signal is subtracted from *S_{back+samp}* signal, obtaining the net contribution of the F-MNP colloidally dispersed to the EMF. This signal (*S_{back+samp} - S_{back}* ) is denominated "*Sₛₐₘₚ*". Subsequently, *Sₛₐₘₚ* is integrated by discrete time steps transforming the EMF voltage units into arbitrary magnetic units. This dynamical magnetic signal (M) is represented versus the magnetic field intensity as a hysteresis loop (see Figure 4B). The M units can be transformed from arbitrary units to magnetic units by comparing the AC magnetic measurement with a quasi-static magnetisation measurement in magnetic units performed by VSM or SQUID at room temperature in the same magnetic field intensity range (see Figure 4C).

Once *S_{back}* is known, the acquisition of "*S_{back+samp}*" is repeated first in absence of the analyte and then, in presence. Thus, AC hysteresis loop of F-MNPs in absence/presence of the analyte is displayed. Preferably, the sample containing the F-MNPs and the analyte to be detected both dispersed in a biological fluid is incubated 1 h at 37°C for allowing the interaction between the F-MNPs and the analyte before being placed into one of the pick-up coil.

The AC magnetisation measurements of apparatus provided by the present invention are fully automatized for 1) sample selection and positioning, 2) generating an alternating magnetic field at given frequency and intensity conditions in order to magnetically excite the F-MNPs, 3) detecting, acquiring and analysing the Electromotive Forces (EMF) induced by F-MNP colloids, 4) extracting the magnetisation as function of the field intensity.

The term "displaying equipment", as used herein, refers to the AC magnetometer that measures the dynamical magnetisation signals of F-MNPs dispersed in aqueous media, such as biological fluids where analyte can be present or absent. The displaying equipment provided by the present application is based on applying the Faraday-Lenz's law of induction under alternating magnetic fields whose frequency ranges between 10 Hz and 1 MHz.

It is believed that this displaying equipment of the apparatus of the invention presents a high sensitivity to the variation of the imaginary part of the magnetic susceptibility due to changes on the relaxation processes after the specific interaction between the receptor and the analyte. The relaxation processes vary when transducer aggregation or volume is altered after the specific interaction with analyte (see figure 1). The sensitivity to measure variations of the dynamical magnetisation signal (i.e. sensor sensitivity) relies on the following parameters:
1) affinity of the interaction between the analyte and the recognition ligands: large values lead to strong interaction between analyte and transducer, favouring the variation of dynamical magnetisation signal,
2) multivalence of the analyte: large values favour transducer agglomeration around the analyte, favouring the increase of hydrodynamic volume of the assembly formed by functionalized magnetic nanoparticles and the analyte and intra-aggregate magnetic dipolar interactions, leading to variation of magnetic relaxation processes, and consequently, of the dynamical magnetisation signal,
3) analyte size: larges values lead to variation of the transducer volume after specific interaction with the analyte, favouring the variation of Brownian relaxation processes, and consequently, the variation of dynamical magnetisation signal,
4) magnetic transducer and analyte concentrations in the biological fluid,
5) dynamical magnetic signal of the magnetic transducer (i.e. relaxation mechanisms and the values of the imaginary part of the dynamical susceptibility of the magnetic nanoparticles employed in the functionalized magnetic nanoparticles), and
6) alternating magnetic field conditions (i.e. values of field frequency and intensity).

One aspect of the invention refers to the use of the apparatus of the invention in the method of the invention for *in vitro* detection of an analyte in aqueous or biological fluids or in the method of the invention for measuring the efficacy of a treatment of a disease in a subject or in the method of the invention of diagnosis of a disease, for measuring the dynamical magnetisation signal of the functionalized magnetic nanoparticles dispersed into the aqueous or biological fluids.

Another aspect of the invention refers to the method of the invention for *in vitro* detection of an analyte in aqueous or biological fluids, wherein the dynamical magnetisation signal of the aqueous or biological fluid is measured with the apparatus above disclosed.

The apparatus of the invention can also be used for *in vitro* detection of variations with time of colloidal stability of the transducer dispersed in different media, such as hydrophilic or hydrophobic media, ion- or protein- rich media, etc. The comparison of the dynamical magnetic signal of the transducer at a given moment with a reference value obtained immediately after its synthesis allows to monitor the variations of the transducer aggregation induced by the dispersion media and/or its surface degradation, influencing their magnetic properties under a alternating magnetic field.

The apparatus of the invention can be also used for measuring the dynamical magnetic signal of functionalized magnetic nanoparticles for its use as industrial quality control to monitor their production and/or preparation of any nanoformulation based on pristine or functionalized magnetic nanoparticles and/or their storage in liquid media. The production of magnetic nanoparticles functionalized with biomolecules to treat a disease, such as drugs, peptides, mRNAs, siRNAs, or coated with any organic molecule in liquid media is susceptible to change the net surface charge along the distinct stages of the functionalization procedure, and therefore, their aggregation degree can change. The latter influences the dynamical magnetic signal, which can be monitored, according to the method of the invention above disclosed, at the end of different functionalization stages.

Another aspect of the invention refers to the method of the invention for measuring the efficacy of a treatment of a disease in a subject, wherein the dynamical magnetisation signal of the biological fluid is measured with the apparatus above disclosed.

Another aspect of the invention refers to the method of the invention of diagnosis of a disease, wherein the dynamical magnetisation signal of the aqueous or biological fluid is measured with the apparatus above disclosed.

### Examples

Specific embodiments of the invention which in no case must be considered limiting are presented below.

The sensor for biomolecular markers based on the variation of the dynamical magnetisation signal of functionalized magnetic nanoparticles after their interaction with an analyte has been tested in the following three examples:

### Example 1: Detection of specific monovalent analyte

In this example magnetic nanoparticles (MNP) are functionalized with the tetratricopeptide (TPR) whose sequence MEEVF is specifically recognized by the VSPₘₒₙₒₘₑᵣ-TPR2-MMY repeat domain (analyte) [Jackrel ME, Valverde R, Regan L. Protein Sci. 2009 18(4):762-74]. Two types of TPR2-MMY modules are used in order to validate the sensor using a monovalent (example 1), and a divalent analyte (example 2).

For this monovalent analyte detection, we employ two MNPs with different magnetic anisotropy values related to the distinct chemical composition (iron oxide and cobalt ferrite) and MNP sizes. The relaxation mechanisms that prevail for each MNP are different, being Néel and Brownian for iron oxide and cobalt ferrite, respectively. Thus, we can compare the efficiency on the dynamical magnetic detection depending on the relaxation mechanisms.

### 1.1. Covalent immobilization of GST-MEEVF onto pre-activated DMSA-MNP and PMAO-MNP

Dimercapto-succinic acid coated magnetic nanoparticles (DMSA-MNP) with an iron oxide magnetic core of 12 nm and poly(maleic anhydride-alt-1-octadecene) coated magnetic nanoparticles (PMAO-MNP) with an iron oxide magnetic core of 21 nm were used in these experiments.

1 mL of DMSA-MNP or PMAO-MNP at 1.5 mg Fe/mL were incubated 1 hour at room temperature with 150 µmol of EDC/g Fe and 75 µmol of NHS/g Fe. Then, the sample was washed by cycles of centrifugation and redispersion in milliQ 10 mM sodium phosphate buffer pH 7.4 (PB buffer) at least 3 times. Then, glutathione S-transferase (GST)-MEEVF was reacted with the activated DMSA-MNP or PMAO-MNP. This pre-activated PMAO-MNP were incubated with 200 µl of GST-MEEVF at 7.5 µM in sodium phosphate buffer 10 mM (PB buffer), 4 h at room temperature and overnight at 4°C. After that, the DMSA-MNP or PMAO-MNP functionalized with GST-MEEVF was purified by filtration thought a sepharose 6 CLB column using PB buffer. Samples of supernatants before and after the immobilization process were extracted and measured using Bradford assay. The number of GST-MEEVF molecules immobilized per DMSA-MNP or PMAO-MNP was 10 molecules (calculated considering the diameter of one DMSA-MNP 12nm or PMAO-MNP 21 nm, as measured by TEM using a JEOL JEM-1010. The amount of bound GST-MEEVF was determined as the difference between the remaining GST-MEEVF concentration in the supernatant at ending of the immobilization process and the GST-MEEVF concentration at the beginning of the immobilization process (µmol GST-MEEVF/mgFe).

### 1.2. Control of the oriented immobilization of GST-MEEVF onto pre-activated DMSA-MNP

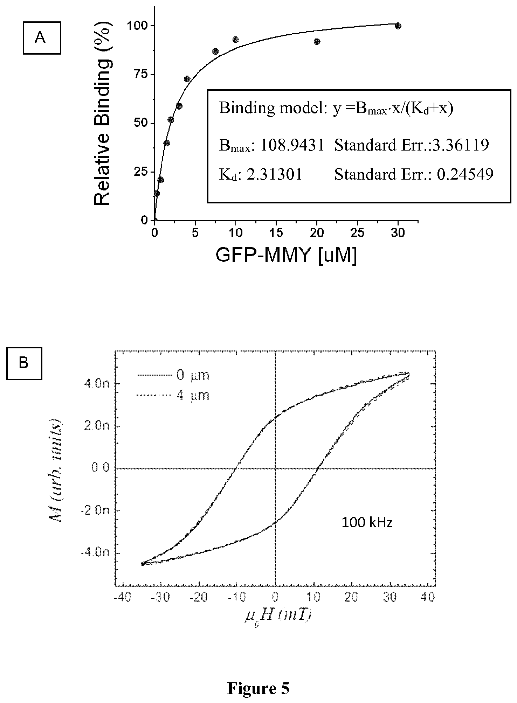

In order to verify the oriented immobilization process of the GST-MEEVF onto the pre-activated DMSA-MNP we have tested the ability of the monomericTPR2-MMY repeat protein to bind the MEEVF peptide fused to a monomeric VFP protein (VFPₘ) or to a dimeric VFP (VFP_{d}) [Ilagan RP, Rhoades E, Gruber DF, Kao HT, Pieribone VA, Regan L. FEBS J. 2010 Apr; 277(8):1967-78]. This interaction has a Kd of 2 µM, therefore if the GST-MEEVF was properly immobilized onto the pre-activated DMSA-MNP, the interaction between DMSA-GST-MEEVF and VFPₘ-TPR2-MMY should have a Kd value around 2 µM. To verify that, DMSA-MNP-GST-MEEVF at 1.5 mg Fe/mL was incubated 30 min at room temperature with 0, 0.5, 1.0, 2.0, 3.0, 4.0, 5.0, 7.5, 10, 20 and 30 µM of VFPₘ-TPR2-MMY. Then, the sample centrifuged 10 min at 21500 rpm and the VFPₘ-TPR2-MMY that is not bound to DMSA-MNP-GST-MEEVF was quantified from the supernatant by UV-visible spectrometry. The amount of VFPₘ-TPR2-MMY bound to DMSA-MNP-GST-MEEVF was determined as the difference between the remaining VFPₘ -TPR2-MMY concentration in the supernatant at ending of the immobilization process and the VFPₘ-TPR2-MMY concentration at the beginning of the immobilization process (relative binding %). Figure 5A shows the relative binding of VFPₘ -TPR2-MMY with DMSA-MNP-GST-MEEVF in function of the VFPₘ-TPR2-MMY concentration. Figure 5A shows that the Kd value is very similar to the Kd value for the interaction between TPR2-MMY and the MEEVF peptide, therefore GST-MEEVF was properly immobilized onto DMSA-MNP.

### 1.3. Magnetic determination of VFPₘ-TPR2-MMY with DMSA-MNP-GST-MEEVF and PMAO-MNP-GST-MEEVF

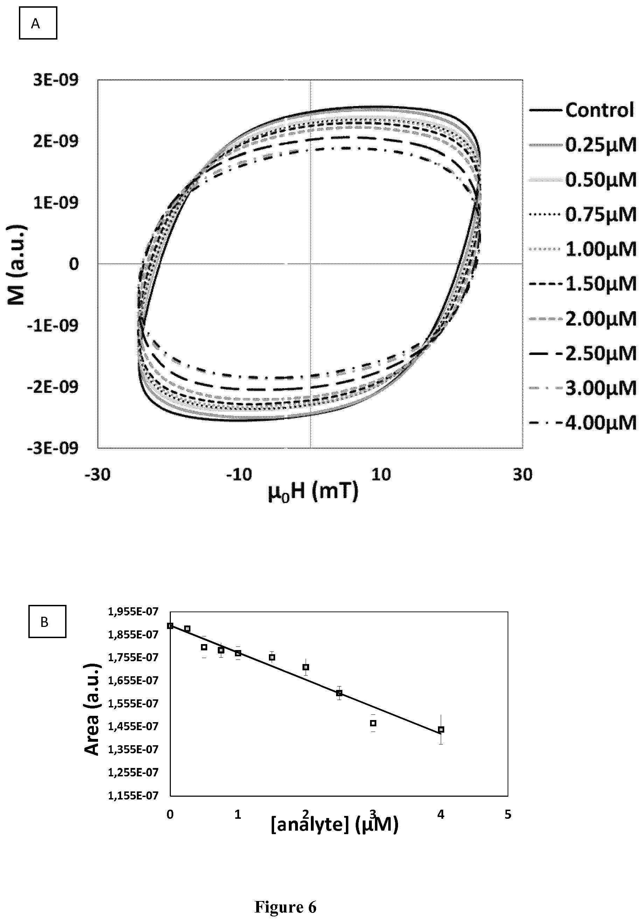

55 µl of DMSA-MNP-GST-MEEVF or PMAO-MNP-GST-MEEVF were incubated 60 minutes at 37°C with 0, 0.25, 0.5, 0.75, 1.0, 1.5, 2.0, 2.5, 3 and 4 µM of VFPₘ -TPR2-MMY in PBS. Then, the hysteresis loops of each sample (DMSA-MNP-GST-MEEVF figure 5B and PMAO-MNP-GST-MEEVF figure 6A) under alternating magnetic fields were measured three times (100 KHz and 34 mT for DMSA-MNP-GST-MEEVF and 50 kHz and 30 mT for PMAO-MNP-GST-MEEVF) with the AC magnetometer of the present invention .

In the case of DMSA-MNP-GST-MEEVF, it could not be detected any decrease of the DMSA-MNP-GST-MEEVF hysteresis loop area when MMY protein was added at the higher concentration (4µm) to a solution containing DMSA-MNP-GST-MEEVF (Figure 5B).

As a first proof-of-concept application, we used the magnetic properties (hysteresis loop area) of the MNP-GST-MEEVF as a sensor to detect the presence of MMY protein. In the case of DMSA-MNP-GST-MEEVF it could not be detected any decrease of the DMSA-MNP-GST-MEEVF hysteresis loop area when MMY protein was added (even at the higher concentration (4µM)) to a solution containing DMSA-MNP-GST-MEEVF (Figure 5B). However, a decrease of the PMAO-MNP-GST-MEEVF hysteresis loop area was detected when MMY protein was added to a solution containing PMAO-MNP-GST-MEEVF (Figure 6A and 6B). The presence of the ligand molecule (VFPₘ-TPR2-MMY) induced a dose-dependent reduction of the hysteresis loop area of the PMAO-MNP-GST-MEEVF related to the variation of the Brownian relaxation process of the F-MNP after interacting with the monovalent analyte (VFPₘ-TPR2-MMY) (Figure 6A). A linear response of hysteresis loop area change was observed in the concentration range of 0.25-4 µM of MMY peptide (Figure 6B).

### Example 2: Detection of specific divalent analyte

In this example magnetic nanoparticles (MNP) are functionalized with the peptide sequence MEEVF that is specifically recognized by the VSP_{dimer}-TPR2-MMY repeat domain (analyte) [Jackrel ME, Valverde R, Regan L. Protein Sci. 2009 18(4):762-74].

For this divalent analyte detection, we employ two MNPs with different magnetic anisotropy values related to the distinct chemical composition (iron oxide and cobalt ferrite) and MNP sizes. The relaxation mechanism that prevails for each MNP is different, being Néel and Brownian for iron oxide and cobalt ferrite, respectively. Thus, we can compare the efficiency on the dynamical magnetic detection depending on the relaxation mechanisms.

### 2.1. Covalent immobilization of GST-MEEVF to pre-activated DMSA-MNP

Dimercapto-succinic acid coated magnetic nanoparticles (DMSA-MNP) with an iron oxide magnetic core of 12 nm were used in these experiments.

1 mL of DMSA-MNP at 1.5 mg Fe/mL were incubated 1 hour at room temperature with 150 µmol of EDC/g Fe and 75 µmol of NHS/g Fe. Then, the sample was washed by cycles of centrifugation and redispersion in milliQ 10 mM sodium phosphate buffer pH 7.4 (PB buffer) at least 3 times. Then, glutathione S-transferase (GST)-MEEVF was reacted with the activated DMSA-MNP. This pre-activated DMSA-MNP were incubated with 200 µl of GST-MEEVF at 7.5 µM in sodium phosphate buffer 10 mM (PB buffer), 4 h at room temperature and overnight at 4°C. After that, the DMSA-MNP functionalized with GST-MEEVF was purified by filtration thought a sepharose 6 CLB column using PB buffer. Samples of supernatants before and after the immobilization process were extracted and measured using Bradford assay. The number of GST-MEEVF molecules immobilized per DMSA-MNP was 10 molecules (calculated considering the diameter of one DMSA-MNP 12 nm, as measured by TEM (JEOL JEM-1010). The amount of bound GST-MEEVF was determined as the difference between the remaining GST-MEEVF concentration in the supernatant at ending of the immobilization process and the GST-MEEVF concentration at the beginning of the immobilization process (µmol GST-MEEVF/mgFe).

### 2.2 Magnetic determination of VFP_{dimer}-TPR2-MMY with DMSA-MNP-GST-MEEVF

55 µl of DMSA-MNP-GST-MEEVF were incubated 60 minutes at 37°C with 0, 0.25, 0.50, 1.0, and 1.5 µM of VFP_{d} -TPR2-MMY in PBS. Then, the hysteresis loops of each sample (figure 2A) under alternating magnetic fields were measured three times (100 kHz and 35 mT) with the AC magnetometer of the present invention.

As a first proof-of-concept application, we used the magnetic properties (hysteresis loop area) of the DMSA-MNP-GST-MEEVF as a sensor to detect the presence of MMY protein (Figure 2A). A decrease of the DMSA-MNP-GST-MEEVF hysteresis loop area was detected when MMY protein was added to a solution containing DMSA-MNP-GST-MEEVF (Figure 2A and 2B). The presence of the ligand molecule (VFP_{d}-TPR2-MMY) induced a dose-dependent reduction of the hysteresis loop area of the DMSA-MNP-GST-MEEVF related to the variation of the Néel relaxation process of the F-MNP induced by magnetic dipolar interaction after the F-MNP entanglement mediated by the multivalent analyte (VFP_{d}-TPR2-MMY) (Figure 2A). A linear response of hysteresis loop area change was observed in the concentration range of 0.25-1.5 µM of MMY peptide (Figure 2B). The DMSA-MNP-GST-MEEVF samples precipitated when the concentration of MMY peptide was over 1.5 µM.

### Example 3: Detection of tetravalent analyte

In this example magnetic nanoparticles (MNP) are functionalized with a GST fusion to the peptide Aceptor peptide (AP) sequence GLNDIFEAQKIEWHE that is biotinylated in the K position (AP-biotin). Biotin is specifically recognized by the tetravalent protein avidin (analyte).

### 3.1. Covalent immobilization of GST-AP-Biotin to pre-activated DMSA-MNP and PMAO-MNP

1 mL of DMSA-MNP or PMAO-MNP at 1.5 mg Fe/mL were incubated 1 hour at room temperature with 150 µmol of EDC/g Fe and 75 µmol of NHS/g Fe. Then, the sample was washed by cycles of centrifugation and redispersion in milliQ 10 mM sodium phosphate buffer pH 7.4 (PB buffer) at least 3 times.

Then, GST-AP-Biotin was reacted with the activated DMSA-MNP or PMAO-MNP. The pre-activated D-MNP or PMAO-MNP were incubated with 200 µl of GST-AP-biotin at 7.5 µM in sodium phosphate buffer 10 mM (PB buffer), 4 h at room temperature and overnight at 4°C. After that, the DMSA-MNP or PMAO-MNP functionalized with GST-AP-biotin was purified by filtration thought a sepharose 6 CLB column using PB buffer. Samples of supernatants before and after the immobilization process were extracted and measured using Bradford assay. The amount of bound GST-MEEVF was determined as the difference between the remaining GST-AP-biotin concentration in the supernatant at ending of the immobilization process and the GST-AP-biotin concentration at the beginning of the immobilization process (µmol GST-AP-biotin/mg Fe). The number of GST-AP-Biotin molecules immobilized per DMSA-MNP or PMAO-MNP was 10 molecules (calculated considering 12 and 21 nm as the diameter of DMSA-MNP or PMAO-MNP, respectively, measured by TEM (JEOL JEM-1010)).

### 3.2 Magnetic determination of avidin with DMSA-MNP-GST-AP-Biotin or PMAO-MNP-GST-AP-Biotin

55 µl of DMSA-MNP-GST-AP-Biotin were incubated 60 minutes at 37°C with 0, 0.083, 0.233, 0.466, 0.70, 0.93 1.17 and 1.6 µM of avidin in PBS. Also, 55 µl of PMAO-MNP-GST-AP-Biotin were incubated 60 minutes at 37°C with 0, 0.025, 0.050, 0.075, 0.100, 0.250, 0.500, 0.750, 1.000 and 1.500 µM of avidin in PBS. Then, the hysteresis loops of each sample under alternating magnetic fields were measured three times (100 kHz and 35 mT for DMSA-MNP-GST-AP-Biotin or 50 kHz and 30 mT for PMAO-MNP-GST-AP-Biotin) with the AC magnetometer of the present invention.

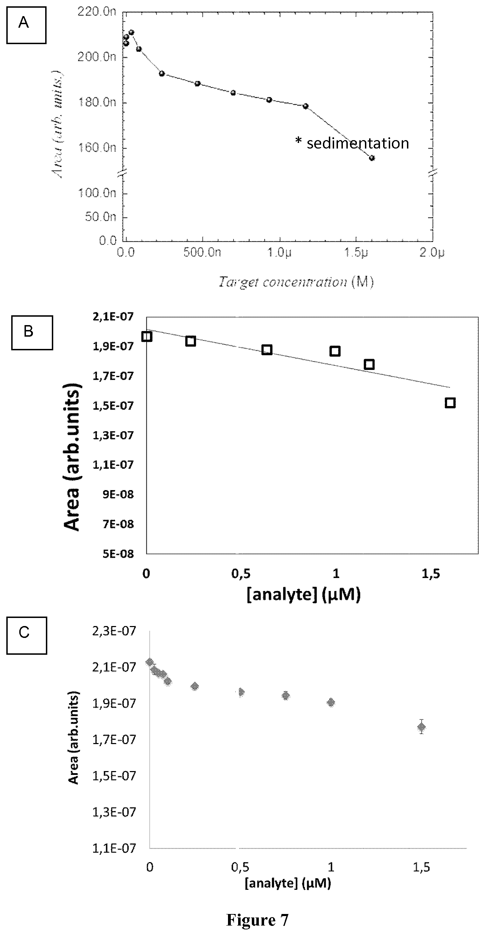

The magnetic properties (hysteresis loop area) of the DMSA-MNP-GST-AP-Biotin and PMAO-MNP-GST-AP-Biotin were used to detect the presence of avidin in PBS. A decrease of the DMSA-MNP-GST-AP-Biotin and PMAO-MNP-GST-AP-Biotin hysteresis loop area was observed when avidin was added to a solution containing DMSA-MNP-GST-AP-Biotin (Figure 7A). The addition of the analyte protein induced a dose-dependent decrease in the hysteresis loop area of the DMSA-MNP-GST-AP-Biotin related to the variation of the Néel relaxation process of the F-MNP induced by magnetic dipolar interaction after the F-MNP entanglement mediated by the specific interaction with avidin. A linear response of hysteresis loop area change was observed in the concentration range of 0.08-1.2 µM of avidin for DMSA-MNP-GST-AP-Biotin (figure 7A). When the concentration of avidin was over 1.2 µM, for DMSA-MNP-GST-AP-Biotin samples precipitated.

In case of the PMAO-MNP-GST-AP-Biotin, the addition of the analyte protein induced a dose-dependent reduction of the hysteresis loop area related to the variation of the Brownian relaxation process of the F-MNP after the specific interaction with avidin. In the case of PMAO-MNP-GST-AP-Biotin, a linear response of hysteresis loop area change was observed in the concentration range of 0.025-1.5 µM of avidin without any sample precipitation up 1.5 µM of avidin (Figure 7C).

Finally, the variation of the dynamical magnetic response (hysteresis loop) of the DMSA-MNP-GST-AP-Biotin was assessed to prove the avidin detection potential in human plasma. A similar decrease of the DMSA-MNP-GST-AP-Biotin hysteresis loop area was observed when avidin was added to a solution containing DMSA-MNP-GST-AP-Biotin (Figure 7B), but with a different reduction rate. The ligand protein induced a dose-dependent decrease in the hysteresis loop area of the DMSA-MNP-GST-AP-Biotin due to the interaction with its cognate protein (avidin). A linear response of hysteresis loop area change was observed in the concentration range of 0.23-1.17 µM of avidin but with a different slope due to the unspecific interaction of human serum proteins with the MNP surface. In spite the MNP surface is not suitable for avoiding such unspecific interacions with serum proteins, it is shown the detection potential of the methodology. At higher concentrations of 1.17 µM of avidin, the DMSA-MNP-GST-AP-Biotin samples precipitate.

In order to verify the use of this detection systems directly in biological samples we have repeat the experiments in human plasma as follows: 55 µl of DMSA-MNP-GST-AP-Biotin were incubated 30 minutes at 37°C with 0, 0.233, 0.70, 0.93, 1.17 and 1.6 µM of avidin in human plasma. Then, the hysteresis loops of each sample under alternating magnetic fields were measured three times (100 kHz and 35 mT) with the AC magnetometer of the present invention.

## Claims

1. Method for *in vitro* detection and/or quantification of an analyte in aqueous or biological fluids comprising:
a) providing functionalized magnetic nanoparticles, wherein each functionalized magnetic nanoparticle comprises a magnetic nanoparticle and a recognition ligand, wherein the magnetic nanoparticle has an average size of from 1 to 100 nm and a saturation magnetisation comprised between 20 and 300 emu/g, and wherein the recognition ligand is linked to said magnetic nanoparticle,
b) incubating the functionalized magnetic nanoparticles of step a) with an aqueous or biological fluid containing the analyte in conditions suitable for producing the binding of said functionalized magnetic nanoparticles to the analyte,
c) measuring the dynamical magnetisation signal of the functionalized magnetic nanoparticles in the aqueous or biological fluid of step b) containing the analyte under an alternating magnetic field, and
d) comparing the dynamical magnetisation signal of the aqueous or biological fluid measured in step c) with a reference value to detect and/or quantify the presence of the analyte in the aqueous o biological fluid; and
wherein the reference value in step d) is that resulting from measuring the dynamical magnetisation signal of the functionalized magnetic nanoparticle of step a) of a reference sample containing the aqueous or biological fluid without the analyte under an alternating magnetic field; and
wherein the dynamical magnetisation signals of steps c) and d) are measured with an apparatus comprising an AC magnetometer; and
wherein the dynamical magnetisation signals are measured as follows:
I. by measuring the induced voltage signals as a function of time under alternating magnetic fields for:
- the functionalized magnetic nanoparticles in the aqueous or biological fluid of step b) containing the analyte under an alternating magnetic field; and/or for
- the functionalized magnetic nanoparticle of step a) of a reference sample containing the aqueous or biological fluid without the analyte under an alternating magnetic field; and
II. by obtaining the hysteresis loop from said induced voltage signals as a function of time for
- the functionalized magnetic nanoparticles in the aqueous or biological fluid of step b) containing the analyte under an alternating magnetic field; and/or for
- the functionalized magnetic nanoparticle of step a) of a reference sample containing the aqueous or biological fluid without the analyte under an alternating magnetic field; and
wherein the comparison of the dynamical magnetisation signal of step d) is performed by comparing the hysteresis loops; preferably by comparing the values of the hysteresis loop parameters obtained from the hysteresis loops; and wherein the dynamical magnetisation signal is measured under alternating magnetic fields at a single resonant frequency.

2. Method according to claim 1, wherein the functionalised magnetic particle is prepared by a process comprising the following steps:
i. activating a magnetic nanoparticle for the immobilisation of a recognition ligand, wherein the activation consists in coating the magnetic nanoparticles with an organic hydrophilic compound; preferably wherein the organic hydrophilic compound contains at least a carboxylic group;
ii. optionally modifying the recognition ligand for the immobilization on the magnetic nanoparticle, and
iii. attaching the recognition ligand of step ii to the magnetic nanoparticle of step i.

3. Method according to any of claims 1 to 2, wherein the magnetic nanoparticle is selected from Fe, Co, Ni, a metal oxide selected from gamma-Fe₂O₃, Fe₃O₄, CoO, Co₃O₄, NiO; a stoichiometric ferrite selected from MnFe₂O₄, CoFe₂O₄, ZnFe₂O₄, NiFe₂O₄, MgFe₂O₄, SrFe₁₂O₁₉ and BaFe₁₂O₁₉; a nonstoichiometric ferrite selected from Fe₃₋ₓMₓO₄, wherein M is a transition element selected from Cr, Mn, Co, Ni and Zn being x >1; MnₐZn₍₁₋ₐ₎Fe₂O₄ and NiₐZn₍₁₋ₐ₎Fe₂O₄ being a<1 and mixtures thereof;
wherein the recognition ligand is selected from a carbohydrate, a peptide, a pseudopeptide, a peptoid, a protein, an antibody, an aptamer, a DNA probe, a RNA probe, a peptide nucleic acid and combinations thereof;
wherein the analyte is either a monovalent or a multivalent analyte, and wherein the recognition ligand onto the functionalised magnetic nanoparticle is either a multivalent recognition ligand or a monovalent recognition ligand; and
wherein the analyte is selected from drugs, doping agents, proteins, peptides, pseudopeptides, nucleic acids, nucleic acid-protein complexes, mRNA, microRNA, lipids, vesicles, vesicle markers, cancerous cell, amino acids, amino acids derivatives, sugars, alkaloids, glycosides, non-ribosomal peptides, phenazines, natural phenols, polyketide, terpenes, and tetrapyrroles.

4. Method according to any of claims 1 to 3, wherein the recognition ligand is a peptide; preferably selected from the group consisting of a cell-penetrating peptide, a signalling peptide and a target binding peptide.

5. Method according to any of claims 1 to 4, wherein the magnetic nanoparticle is a metal oxide selected from gamma-Fe₂O₃ (maghemite), Fe₃O₄(magnetite), CoO, Co₃O₄, and NiO; and
wherein the magnetic nanoparticle is coated with an organic hydrophilic compound containing at least a carboxylic group.

6. Method according to any of claims 1 to 5,
wherein the dynamical magnetisation signal detected under alternating magnetic fields is proportional to the imaginary part of the magnetic susceptibility; and/or
wherein the hysteresis loop is a magnetic hysteresis loop (M) represented versus magnetic field intensity values, wherein said magnetic field intensity values are from -300 kA/m to + 300 kA/m; preferably from -40 kA/m to +40 kA/m.

7. Method according to anyone of claims 1 to 6,
wherein the apparatus comprising an AC magnetometer comprises:
a) an AC magnetic field generator configured to magnetically excite the functionalized magnetic nanoparticles, said AC magnetic field generator comprising a Litz wire coil as an excitation coil, wherein the AC magnetic field generator is part of a LCR circuit allowing to resonantly inject an AC current of a single resonant frequency to the Litz wire coil generating an AC magnetic field wherein the single resonant frequency is within the frequency range from 10 Hz to 1 MHz,
b) a magnetic flux detector comprising two counterwise wounded pick-up coils connected in series and mounted inside the excitation coil, wherein the two pick-up coils have the same turns and dimensions, and
c) a voltage reader,
wherein the voltage reader monitors the voltage signal of the pick-up coils of the magnetic flux detector; and
wherein the excitation coil is immersed into a dielectric liquid flow that circulates across a heat exchanger for thermalization at room temperature.

8. Use of functionalized magnetic nanoparticles for *in vitro* detection and/or quantification of an analyte in aqueous or biological fluids, wherein each functionalized magnetic nanoparticle comprises a magnetic nanoparticle and a recognition ligand, wherein the magnetic nanoparticle has an average size of from 1 to 100 nm and a saturation magnetisation comprised between 20 and 300 emu/g, wherein the recognition ligand is linked to said magnetic nanoparticle, and wherein said analyte is detected in aqueous or biological fluids according to the method as defined in anyone of claims 1 to 7.

9. Method for measuring the efficacy of a treatment of a disease in a subject, comprising:
a) providing functionalized magnetic nanoparticles, wherein each functionalized magnetic nanoparticle comprises a magnetic nanoparticle and a recognition ligand, wherein the magnetic nanoparticle has an average size of from 1 to 100 nm and a saturation magnetisation comprised between 20 and 300 emu/g, and wherein the recognition ligand is linked to said magnetic nanoparticle,
b) incubating the functionalized magnetic nanoparticles of step a) with an aqueous or biological fluid containing an analyte from the subject in conditions suitable for producing the binding of said functionalized magnetic nanoparticles to said analyte,
c) measuring the dynamical magnetisation signal of the functionalized magnetic nanoparticles in the aqueous or biological fluid from the subject of step b) under an alternating magnetic field, and measuring the dynamical magnetisation signal of a reference sample under an alternating magnetic field, wherein said reference sample is obtained from the same subject at an earlier time of point of the disease or prior to the disease, and
d) comparing the dynamical magnetisation signal of the aqueous or biological fluid of the treated subject and that of the reference sample measured in step c), wherein a change of the dynamical magnetic signal of the treated subject with respect to the dynamical magnetic signal of the reference sample is indicative of the efficacy of a treatment of a disease in a subject;
wherein the dynamical magnetisation signals of step c) are measured with an apparatus comprising an AC magnetometer; and
wherein the dynamical magnetisation signal is measured as follows:
I. by measuring the induced voltage signals as a function of time under alternating magnetic fields for:
- the functionalized magnetic nanoparticles in the aqueous or biological fluid of step b) containing the analyte under an alternating magnetic field; and/or for
- the functionalized magnetic nanoparticle of step a) of a reference sample containing the aqueous or biological fluid without the analyte under an alternating magnetic field; and
II. by obtaining the hysteresis loop from said induced voltage signals as a function of time for
- the functionalized magnetic nanoparticles in the aqueous or biological fluid of step b) containing the analyte under an alternating magnetic field; and/or for
- the functionalized magnetic nanoparticle of step a) of a reference sample containing the aqueous or biological fluid without the analyte under an alternating magnetic field;
wherein the comparison of the dynamical magnetisation signal of step d) is performed by comparing the hysteresis loops; preferably by comparing the values of the hysteresis loop parameters obtained from the hysteresis loops; and
wherein the dynamical magnetisation signal is measured under alternating magnetic fields at a single resonant frequency.

10. Use of functionalized magnetic nanoparticles for measuring the efficacy of a treatment of a disease in a subject, wherein each functionalized magnetic nanoparticle comprises a magnetic nanoparticle and a recognition ligand, wherein the magnetic nanoparticle has an average size of from 1 to 100 nm and a saturation magnetisation comprised between 20 and 300 emu/g, wherein the recognition ligand is linked to said magnetic nanoparticle, and wherein the efficacy of said treatment is measured according to the method as defined in claim 9.

11. Use according to claim 10 for measuring the efficacy of a treatment of a disease, wherein the disease is selected from selected from cancer, autoimmune diseases, neurodegenerative diseases, cardiovascular diseases, inflammatory diseases, and endocrine diseases.

12. A method of diagnosis of a disease in a subject comprising the following steps:
a) providing functionalized magnetic nanoparticles, wherein each functionalized magnetic nanoparticle comprises a magnetic nanoparticle and a recognition ligand, wherein the magnetic nanoparticle has an average size of from 1 to 100 nm and a saturation magnetisation comprised between 20 and 300 emu/g, and wherein the recognition ligand is linked to said magnetic nanoparticle,
b) incubating the functionalized magnetic nanoparticles of step a) with an aqueous or biological fluid from the subject in conditions suitable for producing the binding of said functionalized magnetic nanoparticles to an analyte, wherein said analyte is a biomarker of the disease to be diagnosed,
c) measuring the dynamical magnetisation signal of the functionalized magnetic nanoparticles in the aqueous or biological fluid from the subject of step b) under an alternating magnetic field, and
d) comparing the dynamical magnetisation signal of the aqueous or biological fluid of the subject measured in step c) with a reference value indicative of the disease to be diagnosed; and
wherein the dynamical magnetisation signals are measured with an apparatus comprising an AC magnetometer; and
wherein the dynamical magnetisation signal is measured as follows:
I. by measuring the induced voltage signals as a function of time under alternating magnetic fields for:
- the functionalized magnetic nanoparticles in the aqueous or biological fluid of step b) containing the analyte under an alternating magnetic field; and/or for
- the functionalized magnetic nanoparticle of step a) of a reference sample containing the aqueous or biological fluid without the analyte under an alternating magnetic field; and
II. by obtaining the hysteresis loop from said induced voltage signals as a function of time for
- the functionalized magnetic nanoparticles in the aqueous or biological fluid of step b) containing the analyte under an alternating magnetic field; and/or for
- the functionalized magnetic nanoparticle of step a) of a reference sample containing the aqueous or biological fluid without the analyte under an alternating magnetic field; and
wherein the comparison of the dynamical magnetisation signal of step d) is performed by comparing the hysteresis loops; preferably by comparing the values of the hysteresis loop parameters obtained from the hysteresis loops; and
wherein the dynamical magnetisation signal is measured under alternating magnetic fields at a single resonant frequency.

13. Apparatus designed for carrying out the method defined in anyone of claims 1 to 7 or the method according to claim 9 or the method according to claim 12, comprising an AC magnetometer for measuring the dynamical magnetisation signal of functionalized magnetic nanoparticles comprising:
a) an AC magnetic field generator configured to magnetically excite the functionalized magnetic nanoparticles, said AC magnetic field generator comprising a Litz wire coil as an excitation coil, wherein the AC magnetic field generator is part of a LCR circuit allowing to resonantly inject an AC current of a single resonant frequency to the Litz wire coil generating an AC magnetic field wherein the single resonant frequency is within the frequency range from 10 Hz to 1 MHz,
b) a magnetic flux detector comprising two counterwise wounded pick-up coils connected in series and mounted inside the excitation coil, wherein the two pick-up coils have the same turns and dimensions, and
c) a voltage reader,
wherein the voltage reader monitors the voltage signal of the pick-up coils of the magnetic flux detector; and
wherein the excitation coil is immersed into a dielectric liquid flow that circulates across a heat exchanger for thermalization at room temperature.

14. Use of the apparatus according to claim 13 in the method according to claims 1 to 7 or in the method according to claim 9 or in the method according to claim 12, for measuring the dynamical magnetisation signal of the functionalized magnetic nanoparticles dispersed into the aqueous or biological fluids.

15. *In vitro* use of functionalized magnetic nanoparticles for diagnosing a disease, wherein each functionalised magnetic nanoparticle comprises a magnetic nanoparticle and a recognition ligand, wherein the magnetic nanoparticle has an average size of from about 1 to about 100 nm and a saturation magnetisation comprised between 20 and 300 emu/g, wherein the recognition ligand is linked to said magnetic nanoparticle, and wherein the disease is diagnosed by *in vitro* detection and/or quantification of an analyte in aqueous or biological fluids according to the method as defined in anyone of claims 1 to 6; and optionally, wherein the disease is selected from cancer, autoimmune diseases, neurodegenerative diseases, cardiovascular diseases, inflammatory diseases, and endocrine diseases.

## Patentansprüche

1. Verfahren zum In-vitro-Nachweis und/oder zur Quantifizierung eines Analyten in wässrigen oder biologischen Flüssigkeiten, umfassend:
a) Bereitstellen funktionalisierter magnetischer Nanopartikel, wobei jedes funktionalisierte magnetische Nanopartikel ein magnetisches Nanopartikel und einen Erkennungsliganden umfasst, wobei das magnetische Nanopartikel eine durchschnittliche Größe von 1 bis 100 nm und eine Sättigungsmagnetisierung zwischen 20 und 300 emu/g umfasst und wobei der Erkennungsligand an das magnetische Nanopartikel gebunden ist,
b) Inkubieren der in Schritt a) funktionalisierten magnetischen Nanopartikel mit einer wässrigen oder biologischen Flüssigkeit, die den Analyten enthält, unter Bedingungen, die geeignet sind, die Bindung der funktionalisierten magnetischen Nanopartikel an den Analyten zu bewirken,
c) Messen des dynamischen Magnetisierungssignals der funktionalisierten magnetischen Nanopartikel in der wässrigen oder biologischen Flüssigkeit aus Schritt b), die den Analyten enthält, unter einem alternierenden Magnetfeld, und
d) Vergleichen des in Schritt c) gemessenen dynamischen Magnetisierungssignals der wässrigen oder biologischen Flüssigkeit mit einem Referenzwert, um das Vorhandensein des Analyten in der wässrigen oder biologischen Flüssigkeit nachzuweisen und/oder zu quantifizieren; und
wobei der Referenzwert in Schritt d) derjenige ist, der sich aus der Messung des dynamischen Magnetisierungssignals des funktionalisierten magnetischen Nanopartikels aus Schritt a) einer Referenzprobe, die die wässrige oder biologische Flüssigkeit ohne den Analyten enthält, unter einem alternierenden Magnetfeld ergibt; und
wobei die dynamischen Magnetisierungssignale der Schritte c) und d) mit einer Vorrichtung gemessen werden, die ein Wechselstrommagnetometer umfasst; und
wobei die dynamischen Magnetisierungssignale wie folgt gemessen werden:
I. durch Messung der induzierten Spannungssignale als Funktion der Zeit unter alternierenden Magnetfeldern für:
- die funktionalisierten magnetischen Nanopartikel in der wässrigen oder biologischen Flüssigkeit aus Schritt b), die den Analyten enthält, unter einem alternierenden Magnetfeld; und/oder für
- das funktionalisierte magnetische Nanopartikel aus Schritt a) einer Referenzprobe, die die wässrige oder biologische Flüssigkeit ohne den Analyten enthält, unter einem alternierenden Magnetfeld; und
II. durch Ermittlung der Hystereseschleife aus den genannten induzierten Spannungssignalen als Funktion der Zeit für
- die funktionalisierten magnetischen Nanopartikel in der wässrigen oder biologischen Flüssigkeit aus Schritt b), die den Analyten enthält, unter einem alternierenden Magnetfeld; und/oder für
- das funktionalisierte magnetische Nanopartikel aus Schritt a) einer Referenzprobe, die die wässrige oder biologische Flüssigkeit ohne den Analyten enthält, unter einem alternierenden Magnetfeld; und
wobei der Vergleich des dynamischen Magnetisierungssignals aus Schritt d) durch Vergleich der Hystereseschleifen erfolgt; vorzugsweise durch Vergleich der aus den Hystereseschleifen gewonnenen Werte der Hystereseschleifenparameter; und
wobei das dynamische Magnetisierungssignal unter alternierenden Magnetfeldern bei einer einzigen Resonanzfrequenz gemessen wird.

2. Verfahren nach Anspruch 1, wobei das funktionalisierte Magnetpartikel durch ein Verfahren hergestellt wird, das die folgenden Schritte umfasst:
i. Aktivieren eines magnetischen Nanopartikels zur Immobilisierung eines Erkennungsliganden, wobei die Aktivierung in dem Beschichten der magnetischen Nanopartikel mit einer organischen hydrophilen Verbindung besteht; vorzugsweise wobei die organische hydrophile Verbindung mindestens eine Carboxylgruppe enthält;
ii. gegebenenfalls Modifizieren des Erkennungsliganden für die Immobilisierung auf dem magnetischen Nanopartikel, und
iii. Anbringen des Erkennungsliganden aus Schritt ii an das magnetische Nanopartikel aus Schritt i.

3. Verfahren nach einem der Ansprüche 1 oder 2, wobei das magnetische Nanopartikel ausgewählt ist aus Fe, Co, Ni, einem Metalloxid ausgewählt aus gamma-Fe₂O₃, Fe₃O₄, CoO, Co₃O₄, NiO; einem stöchiometrischen Ferrit ausgewählt aus MnFe₂O₄, CoFe₂O₄, ZnFe₂O₄, NiFe₂O₄, MgFe₂O₄, SrFe₁₂O₁₉ und BaFe₁₂O₁₉; einem nichtstöchiometrischen Ferrit ausgewählt aus Fe₃₋ₓMₓO₄, wobei M ein Übergangsmetall ausgewählt aus Cr, Mn, Co, Ni und Zn mit x > 1 ist; MnₐZn₍₁₋ₐ₎Fe₂O₄ und NiₐZn₍₁₋ₐ₎Fe₂O₄ mit a < 1 und Gemischen davon; wobei der Erkennungsligand ausgewählt ist aus einem Kohlenhydrat, einem Peptid, einem Pseudopeptid, einem Peptoid, einem Protein, einem Antikörper, einem Aptamer, einer DNA-Sonde, einer RNA-Sonde, einer Peptidnukleinsäure und Kombinationen davon;
wobei es sich bei dem Analyten entweder um einen monovalenten oder einen multivalenten Analyten handelt und wobei der Erkennungsligand auf dem funktionalisierten magnetischen Nanopartikel entweder ein multivalenter oder ein monovalenter Erkennungsligand ist; und wobei der Analyt ausgewählt ist aus Arzneimitteln, Dopingmitteln, Proteinen, Peptiden, Pseudopeptiden, Nukleinsäuren, Nukleinsäure-Protein-Komplexen, mRNA, microRNA, Lipiden, Vesikeln, Vesikelmarkern, Krebszellen, Aminosäuren, Aminosäurederivaten, Zuckern, Alkaloiden, Glykosiden, nichtribosomalen Peptiden, Phenazinen, natürlichen Phenolen, Polyketiden, Terpenen und Tetrapyrrolen.

4. Verfahren nach einem der Ansprüche 1 bis 3, wobei der Erkennungsligand ein Peptid ist; vorzugsweise ausgewählt aus der Gruppe, bestehend aus einem zellpenetrierenden Peptid, einem Signalpeptid und einem zielbindenden Peptid.

5. Verfahren nach einem der Ansprüche 1 bis 4, wobei es sich bei dem magnetischen Nanopartikel um ein Metalloxid handelt, ausgewählt aus gamma-Fe₂O₃ (Maghemit), Fe₃O₄ (Magnetit), CoO, Co₃O₄ und NiO; und
wobei das magnetische Nanopartikel mit einer organischen hydrophilen Verbindung beschichtet ist, die mindestens eine Carboxylgruppe enthält.

6. Verfahren nach einem der Ansprüche 1 bis 5, wobei das unter alternierenden Magnetfeldern erfasste dynamische Magnetisierungssignal proportional zum Imaginärteil der magnetischen Suszeptibilität ist; und / oder
wobei es sich bei der Hystereseschleife um eine magnetische Hystereseschleife (M) handelt, die in Abhängigkeit von den Magnetfeldstärkewerten dargestellt ist, wobei die Magnetfeldstärkewerte von -300 kA/m bis +300 kA/ m, vorzugsweise von -40 kA/m bis +40 kA/m, liegen.

7. Verfahren gemäß einem der Ansprüche 1 bis 6,
wobei die Vorrichtung, die ein Wechselstrommagnetometer umfasst, Folgendes umfasst:
a) einen Wechselfeldgenerator, der so konfiguriert ist, dass er die funktionalisierten magnetischen Nanopartikel magnetisch anregt, wobei der Wechselfeldgenerator eine Litzendrahtspule als Erregerspule umfasst und Teil eines LCR-Schwingkreises ist, der es ermöglicht, einen Wechselstrom mit einer einzigen Resonanzfrequenz resonant in die Litzendrahtspule einzuspeisen und so ein Wechselfeld zu erzeugen, dessen Resonanzfrequenz im Frequenzbereich von 10 Hz bis 1 MHz liegt,
b) einen Magnetflussdetektor mit zwei gegenläufig gewickelten, in Reihe geschalteten und innerhalb der Erregerspule montierten Aufnahmespulen, wobei die beiden Aufnahmespulen die gleiche Windungszahl und Abmessungen aufweisen, und
c) ein Spannungsmessgerät,
wobei das Spannungsmessgerät das Spannungssignal der Aufnahmespulen des Magnetflussdetektors überwacht; und
wobei die Erregerspule in einen dielektrischen Flüssigkeitsstrom eingetaucht ist, der zur Thermalisierung bei Raumtemperatur über einen Wärmetauscher zirkuliert.

8. Verwendung von funktionalisierten magnetischen Nanopartikeln zum *In-vitro-*Nachweis und/oder zur Quantifizierung eines Analyten in wässrigen oder biologischen Flüssigkeiten, wobei jedes funktionalisierte magnetische Nanopartikel ein magnetisches Nanopartikel und einen Erkennungsliganden umfasst, wobei das magnetische Nanopartikel eine durchschnittliche Größe von 1 bis 100 nm und eine Sättigungsmagnetisierung zwischen 20 und 300 emu/g umfasst und der Erkennungsligand an das magnetische Nanopartikel gebunden ist,
und wobei der Analyt in wässrigen oder biologischen Flüssigkeiten nach dem in einem der Ansprüche 1 bis 7 definierten Verfahren nachgewiesen wird.

9. Verfahren zur Messung der Wirksamkeit einer Behandlung einer Krankheit bei einem Probanden, umfassend:
a) Bereitstellen funktionalisierter magnetischer Nanopartikel, wobei jedes funktionalisierte magnetische Nanopartikel ein magnetisches Nanopartikel und einen Erkennungsliganden umfasst, wobei das magnetische Nanopartikel eine durchschnittliche Größe von 1 bis 100 nm und eine Sättigungsmagnetisierung zwischen 20 und 300 emu/g umfasst und wobei der Erkennungsligand an das magnetische Nanopartikel gebunden ist,
b) Inkubieren der in Schritt a) funktionalisierten magnetischen Nanopartikel mit einer wässrigen oder biologischen Flüssigkeit, die einen Analyten des Probanden enthält, unter Bedingungen, die geeignet sind, die Bindung der funktionalisierten magnetischen Nanopartikel an den Analyten zu bewirken,
c) Messen des dynamischen Magnetisierungssignals der funktionalisierten magnetischen Nanopartikel in der wässrigen oder biologischen Flüssigkeit des Probanden aus Schritt b) unter einem alternierenden Magnetfeld und Messen des dynamischen Magnetisierungssignals einer Referenzprobe unter einem alternierenden Magnetfeld, wobei die Referenzprobe vom selben Probanden zu einem früheren Zeitpunkt der Erkrankung oder vor deren Ausbruch gewonnen wurde, und
d) Vergleichen des dynamischen Magnetisierungssignals der wässrigen oder biologischen Flüssigkeit des behandelten Probanden mit dem der in Schritt c) gemessenen Referenzprobe, wobei eine Änderung des dynamischen Magnetisierungssignals des behandelten Probanden im Vergleich zum dynamischen Magnetisierungssignal der Referenzprobe ein Indikator für die Wirksamkeit einer Behandlung einer Krankheit bei einem Probanden ist;
wobei die dynamischen Magnetisierungssignale aus Schritt c) mit einer Vorrichtung gemessen werden, die ein Wechselstrommagnetometer umfasst; und
wobei das dynamische Magnetisierungssignal wie folgt gemessen wird:
I. durch Messung der induzierten Spannungssignale als Funktion der Zeit unter alternierenden Magnetfeldern für:
- die funktionalisierten magnetischen Nanopartikel in der wässrigen oder biologischen Flüssigkeit aus Schritt b), die den Analyten enthält, unter einem alternierenden Magnetfeld; und/oder für
- das funktionalisierte magnetische Nanopartikel aus Schritt a) einer Referenzprobe, die die wässrige oder biologische Flüssigkeit ohne den Analyten enthält, unter einem alternierenden Magnetfeld; und
II. durch Ermittlung der Hystereseschleife aus den genannten induzierten Spannungssignalen als Funktion der Zeit für
- die funktionalisierten magnetischen Nanopartikel in der wässrigen oder biologischen Flüssigkeit aus Schritt b), die den Analyten enthält, unter einem alternierenden Magnetfeld; und/oder für
- das funktionalisierte magnetische Nanopartikel aus Schritt a) einer Referenzprobe, die die wässrige oder biologische Flüssigkeit ohne den Analyten enthält, unter einem alternierenden Magnetfeld;
wobei der Vergleich des dynamischen Magnetisierungssignals aus Schritt d) durch Vergleich der Hystereseschleifen erfolgt; vorzugsweise durch Vergleich der aus den Hystereseschleifen gewonnenen Werte der Hystereseschleifenparameter; und
wobei das dynamische Magnetisierungssignal unter alternierenden Magnetfeldern bei einer einzigen Resonanzfrequenz gemessen wird.

10. Verwendung von funktionalisierten magnetischen Nanopartikeln zur Messung der Wirksamkeit einer Behandlung einer Krankheit bei einem Probanden, wobei jeder funktionalisierte magnetische Nanopartikel ein magnetisches Nanopartikel und einen Erkennungsliganden umfasst,
wobei das magnetische Nanopartikel eine durchschnittliche Größe von 1 bis 100 nm und eine Sättigungsmagnetisierung zwischen 20 und 300 emu/g umfasst, wobei der Erkennungsligand an das magnetische Nanopartikel gebunden ist und die Wirksamkeit der Behandlung nach dem in Anspruch 9 definierten Verfahren gemessen wird.

11. Verwendung nach Anspruch 10 zur Messung der Wirksamkeit einer Behandlung einer Krankheit, wobei die Krankheit ausgewählt ist aus ausgewählt aus Krebs, Autoimmunerkrankungen, neurodegenerativen Erkrankungen, Herz-Kreislauf-Erkrankungen, entzündlichen Erkrankungen und endokrinen Erkrankungen.

12. Ein Verfahren zur Diagnose einer Krankheit bei einem Probanden, umfassend die folgenden Schritte:
a) Bereitstellen funktionalisierter magnetischer Nanopartikel, wobei jedes funktionalisierte magnetische Nanopartikel ein magnetisches Nanopartikel und einen Erkennungsliganden umfasst, wobei das magnetische Nanopartikel eine durchschnittliche Größe von 1 bis 100 nm und eine Sättigungsmagnetisierung zwischen 20 und 300 emu/g umfasst und wobei der Erkennungsligand an das magnetische Nanopartikel gebunden ist,
b) Inkubieren der in Schritt a) funktionalisierten magnetischen Nanopartikel mit einer wässrigen oder biologischen Flüssigkeit des Probanden unter Bedingungen, die geeignet sind, die Bindung der funktionalisierten magnetischen Nanopartikel an einen Analyten zu bewirken, wobei es sich bei dem Analyten um einen Biomarker der zu diagnostizierenden Krankheit handelt,
c) Messen des dynamischen Magnetisierungssignals der funktionalisierten magnetischen Nanopartikel in der wässrigen oder biologischen Flüssigkeit des in Schritt b) untersuchten Probanden unter einem alternierenden Magnetfeld, und
d) Vergleichen des in Schritt c) gemessenen dynamischen Magnetisierungssignals der wässrigen oder biologischen Flüssigkeit des Probanden mit einem Referenzwert, der auf die zu diagnostizierende Krankheit hinweist; und
wobei die dynamischen Magnetisierungssignale mit einer Vorrichtung gemessen werden, die ein Wechselstrommagnetometer umfasst; und
wobei das dynamische Magnetisierungssignal wie folgt gemessen wird:
I. durch Messung der induzierten Spannungssignale als Funktion der Zeit unter alternierenden Magnetfeldern für:
- die funktionalisierten magnetischen Nanopartikel in der wässrigen oder biologischen Flüssigkeit aus Schritt b), die den Analyten enthält, unter einem alternierenden Magnetfeld; und/oder für
- das funktionalisierte magnetische Nanopartikel aus Schritt a) einer Referenzprobe, die die wässrige oder biologische Flüssigkeit ohne den Analyten enthält, unter einem alternierenden Magnetfeld; und
II. durch Ermittlung der Hystereseschleife aus den genannten induzierten Spannungssignalen als Funktion der Zeit für
- die funktionalisierten magnetischen Nanopartikel in der wässrigen oder biologischen Flüssigkeit aus Schritt b), die den Analyten enthält, unter einem alternierenden Magnetfeld; und/oder für
- das funktionalisierte magnetische Nanopartikel aus Schritt a) einer Referenzprobe, die die wässrige oder biologische Flüssigkeit ohne den Analyten enthält, unter einem alternierenden Magnetfeld; und
wobei der Vergleich des dynamischen Magnetisierungssignals aus Schritt d) durch Vergleich der Hystereseschleifen erfolgt; vorzugsweise durch Vergleich der aus den Hystereseschleifen gewonnenen Werte der Hystereseschleifenparameter; und wobei das dynamische Magnetisierungssignal unter alternierenden Magnetfeldern bei einer einzigen Resonanzfrequenz gemessen wird.

13. Vorrichtung zur Durchführung des Verfahrens nach einem der Ansprüche 1 bis 7 oder des Verfahrens nach Anspruch 9 oder des Verfahrens nach Anspruch 12, umfassend ein Wechselstrommagnetometer zur Messung des dynamischen Magnetisierungssignals von funktionalisierten magnetischen Nanopartikeln, umfassend:
a) einen Wechselfeldgenerator, der so konfiguriert ist, dass er die funktionalisierten magnetischen Nanopartikel magnetisch anregt, wobei der Wechselfeldgenerator eine Litzendrahtspule als Erregerspule umfasst und Teil eines LCR-Schwingkreises ist, der es ermöglicht, einen Wechselstrom mit einer einzigen Resonanzfrequenz resonant in die Litzendrahtspule einzuspeisen und so ein Wechselfeld zu erzeugen, dessen Resonanzfrequenz im Frequenzbereich von 10 Hz bis 1 MHz liegt,
b) einen Magnetflussdetektor mit zwei gegenläufig gewickelten, in Reihe geschalteten und innerhalb der Erregerspule montierten Aufnahmespulen, wobei die beiden Aufnahmespulen die gleichen Windungen und Abmessungen aufweisen, und
c) ein Spannungsmessgerät,
wobei das Spannungsmessgerät das Spannungssignal der Aufnahmespulen des Magnetflussdetektors überwacht; und
wobei die Erregerspule in einen dielektrischen Flüssigkeitsstrom eingetaucht ist, der zur Thermalisierung bei Raumtemperatur über einen Wärmetauscher zirkuliert.

14. Verwendung der Vorrichtung nach Anspruch 13 in dem Verfahren nach den Ansprüchen 1 bis 7 oder in dem Verfahren nach Anspruch 9 oder in dem Verfahren nach Anspruch 12 zur Messung des dynamischen Magnetisierungssignals der in wässrigen oder biologischen Flüssigkeiten dispergierten funktionalisierten magnetischen Nanopartikel.

15. In-vitro-Anwendung funktionalisierter magnetischer Nanopartikel zur Diagnose einer Krankheit, wobei jedes funktionalisierte magnetische Nanopartikel ein magnetisches Nanopartikel und einen Erkennungsliganden umfasst, wobei das magnetische Nanopartikel eine durchschnittliche Größe von etwa 1 bis etwa 100 nm und eine Sättigungsmagnetisierung zwischen 20 und 300 emu/g umfasst, wobei der Erkennungsligand an das magnetische Nanopartikel gebunden ist und wobei die Krankheit durch *In-vitro-Nachweis* und/oder -Quantifizierung eines Analyten in wässrigen oder biologischen Flüssigkeiten gemäß dem in einem der Ansprüche 1 bis 6 definierten Verfahren diagnostiziert wird; und optional, wobei die Krankheit ausgewählt ist aus Krebs, Autoimmunerkrankungen, neurodegenerativen Erkrankungen, Herz-Kreislauf-Erkrankungen, entzündlichen Erkrankungen und endokrinen Erkrankungen.

## Revendications

1. Procédé de détection et/ou quantification *in vitro* d'un analyte dans des fluides aqueux ou biologiques comprenant :
a) la fourniture de nanoparticules magnétiques fonctionnalisées, dans lequel chaque nanoparticule magnétique fonctionnalisée comprend une nanoparticule magnétique et un ligand de reconnaissance, dans lequel la nanoparticule magnétique a une taille moyenne de 1 à 100 nm et une aimantation à saturation comprise entre 20 et 300 emu/g, et dans lequel le ligand de reconnaissance est lié à ladite nanoparticule magnétique,
b) l'incubation des nanoparticules magnétiques fonctionnalisées de l'étape a) avec un fluide aqueux ou biologique contenant l'analyte dans des conditions appropriées pour produire la liaison desdites nanoparticules magnétiques fonctionnalisées à l'analyte,
c) la mesure du signal d'aimantation dynamique des nanoparticules magnétiques fonctionnalisées dans le fluide aqueux ou biologique de l'étape b) contenant l'analyte sous un champ magnétique alternatif, et
d) la comparaison du signal d'aimantation dynamique du fluide aqueux ou biologique mesuré à l'étape c) à une valeur de référence pour détecter et/ou quantifier la présence de l'analyte dans le fluide aqueux ou biologique ; et dans lequel la valeur de référence à l'étape d) est celle résultant de la mesure du signal d'aimantation dynamique de la nanoparticule magnétique fonctionnalisée de l'étape a) d'un échantillon de référence contenant le fluide aqueux ou biologique sans l'analyte sous un champ magnétique alternatif ; et
dans lequel les signaux d'aimantation dynamique des étapes c) et d) sont mesurés avec un appareil comprenant un magnétomètre CA ; et
dans lequel les signaux d'aimantation dynamique sont mesurés comme suit :
I. en mesurant les signaux de tension induits en fonction du temps sous des champs magnétiques alternatifs pour :
- les nanoparticules magnétiques fonctionnalisées dans le fluide aqueux ou biologique de l'étape b) contenant l'analyte sous un champ magnétique alternatif ; et/ou pour
- la nanoparticule magnétique fonctionnalisée de l'étape a) d'un échantillon de référence contenant le fluide aqueux ou biologique sans l'analyte sous un champ magnétique alternatif ; et
II. en obtenant la boucle d'hystérésis à partir desdits signaux de tension induits en fonction du temps pour
- les nanoparticules magnétiques fonctionnalisées dans le fluide aqueux ou biologique de l'étape b) contenant l'analyte sous un champ magnétique alternatif ; et/ou pour
- la nanoparticule magnétique fonctionnalisée de l'étape a) d'un échantillon de référence contenant le fluide aqueux ou biologique sans l'analyte sous un champ magnétique alternatif ; et
dans lequel la comparaison du signal d'aimantation dynamique de l'étape d) est réalisée en comparant les boucles d'hystérésis ; de préférence en comparant les valeurs des paramètres de boucle d'hystérésis obtenus à partir des boucles d'hystérésis ; et
dans lequel le signal d'aimantation dynamique est mesuré sous des champs magnétiques alternatifs à une seule fréquence de résonance.

2. Procédé selon la revendication 1, dans lequel la particule magnétique fonctionnalisée est préparée par un procédé comprenant les étapes suivantes :
i. l'activation d'une nanoparticule magnétique pour l'immobilisation d'un ligand de reconnaissance, dans lequel l'activation consiste à enrober les nanoparticules magnétiques d'un composé hydrophile organique ; de préférence dans lequel le composé hydrophile organique contient au moins un groupe carboxylique ;
ii. la modification facultative du ligand de reconnaissance pour l'immobilisation sur la nanoparticule magnétique, et
iii. la fixation du ligand de reconnaissance de l'étape ii à la nanoparticule magnétique de l'étape i.

3. Procédé selon l'une quelconque des revendications 1 à 2, dans lequel la nanoparticule magnétique est sélectionnée parmi Fe, Co, Ni, un oxyde métallique sélectionné parmi gamma-Fe₂O₃, Fe₃O₄, CoO, Co₃O₄, NiO ; un ferrite stœchiométrique sélectionné parmi MnFe₂O₄, CoFe₂O₄, ZnFe₂O₄, NiFe₂O₄, MgFe₂O₄, SrFe₁₂O₁₉ et BaFe₁₂O₁₉ ; un ferrite non stœchiométrique sélectionné parmi Fe₃₋ₓMₓO₄, dans lequel M est un élément de transition sélectionné parmi Cr, Mn, Co, Ni et Zn étant x > 1 ; MnₐZn₍₁₋ₐ₎Fe₂O₄ et NiₐZn₍₁₋ₐ₎Fe₂O₄ étant a < 1 et leurs mélanges ;
dans lequel le ligand de reconnaissance est sélectionné parmi un glucide, un peptide, un pseudopeptide, un peptoïde, une protéine, un anticorps, un aptamère, une sonde ADN, une sonde ARN, un acide nucléique peptidique et leurs combinaisons ;
dans lequel l'analyte est soit un analyte monovalent, soit un analyte multivalent, et dans lequel le ligand de reconnaissance sur la nanoparticule magnétique fonctionnalisée est soit un ligand de reconnaissance multivalent, soit un ligand de reconnaissance monovalent ; et
dans lequel l'analyte est sélectionné parmi les médicaments, agents dopants, protéines, peptides, pseudopeptides, acides nucléiques, complexes acide nucléique-protéine, ARNm, microARN, lipides, vésicules, marqueurs de vésicules, cellule cancéreuse, acides aminés, dérivés d'acides aminés, sucres, alcaloïdes, glycosides, peptides non ribosomiques, phénazines, phénols naturels, polycétides, terpènes et tétrapyrroles.

4. Procédé selon l'une quelconque des revendications 1 à 3, dans lequel le ligand de reconnaissance est un peptide ; de préférence sélectionné dans le groupe consistant en un peptide de pénétration cellulaire, un peptide de signalisation et un peptide de liaison cible.

5. Procédé selon l'une quelconque des revendications 1 à 4, dans lequel la nanoparticule magnétique est un oxyde métallique sélectionné parmi gamma-Fe₂O₃ (maghémite), Fe₃O₄ (magnétite), CoO, Co₃O₄ et NiO ; et
dans lequel la nanoparticule magnétique est enrobée d'un composé hydrophile organique contenant au moins un groupe carboxylique.

6. Procédé selon l'une quelconque des revendications 1 à 5,
dans lequel le signal d'aimantation dynamique détecté sous des champs magnétiques alternatifs est proportionnel à la partie imaginaire de la susceptibilité magnétique ; et/ou
dans lequel la boucle d'hystérésis est une boucle d'hystérésis magnétique (M) représentée par rapport à des valeurs d'intensité de champ magnétique, dans lequel lesdites valeurs d'intensité de champ magnétique vont de -300 kA/m à +300 kA/m ; de préférence de -40 kA/m à +40 kA/m.

7. Procédé selon l'une quelconque des revendications 1 à 6,
dans lequel l'appareil comprenant un magnétomètre CA comprend :
a) un générateur de champ magnétique CA configuré pour exciter magnétiquement les nanoparticules magnétiques fonctionnalisées, ledit générateur de champ magnétique CA comprenant une bobine de fil de Litz en tant que bobine d'excitation, dans lequel le générateur de champ magnétique CA fait partie d'un circuit LCR permettant d'injecter de manière résonante un courant CA d'une fréquence de résonance unique dans la bobine de fil de Litz générant un champ magnétique CA dans lequel la fréquence de résonance unique est dans la plage de fréquences de 10 Hz à 1 MHz,
b) un détecteur de flux magnétique comprenant deux bobines exploratrices enroulées en sens inverse reliées en série et montées à l'intérieur de la bobine d'excitation, dans lequel les deux bobines exploratrices présentent les mêmes spires et dimensions, et
c) un lecteur de tension,
dans lequel le lecteur de tension surveille le signal de tension des bobines exploratrices du détecteur de flux magnétique ; et
dans lequel la bobine d'excitation est immergée dans un flux de liquide diélectrique qui circule à travers un échangeur de chaleur pour une thermalisation à température ambiante.

8. Utilisation de nanoparticules magnétiques fonctionnalisées pour la détection et/ou la quantification *in vitro* d'un analyte dans des fluides aqueux ou biologiques, dans laquelle chaque nanoparticule magnétique fonctionnalisée comprend une nanoparticule magnétique et un ligand de reconnaissance, dans laquelle la nanoparticule magnétique a une taille moyenne de 1 à 100 nm et une aimantation à saturation comprise entre 20 et 300 emu/g, dans laquelle le ligand de reconnaissance est lié à ladite nanoparticule magnétique, et dans laquelle ledit analyte est détecté dans des fluides aqueux ou biologiques selon le procédé défini dans l'une quelconque des revendications 1 à 7.

9. Procédé de mesure de l'efficacité d'un traitement d'une maladie chez un sujet, comprenant :
a) la fourniture de nanoparticules magnétiques fonctionnalisées, dans lequel chaque nanoparticule magnétique fonctionnalisée comprend une nanoparticule magnétique et un ligand de reconnaissance, dans lequel la nanoparticule magnétique a une taille moyenne de 1 à 100 nm et une aimantation à saturation comprise entre 20 et 300 emu/g, et dans lequel le ligand de reconnaissance est lié à ladite nanoparticule magnétique,
b) l'incubation des nanoparticules magnétiques fonctionnalisées de l'étape a) avec un fluide aqueux ou biologique contenant un analyte provenant du sujet dans des conditions appropriées pour produire la liaison desdites nanoparticules magnétiques fonctionnalisées audit analyte,
c) la mesure du signal d'aimantation dynamique des nanoparticules magnétiques fonctionnalisées dans le fluide aqueux ou biologique provenant du sujet de l'étape b) sous un champ magnétique alternatif, et la mesure du signal d'aimantation dynamique d'un échantillon de référence sous un champ magnétique alternatif, dans lequel ledit échantillon de référence est obtenu auprès du même sujet à un moment antérieur à la maladie ou avant la maladie, et
d) la comparaison du signal d'aimantation dynamique du fluide aqueux ou biologique du sujet traité et de celui de l'échantillon de référence mesurés à l'étape c), dans lequel un changement du signal magnétique dynamique du sujet traité par rapport au signal magnétique dynamique de l'échantillon de référence indique l'efficacité d'un traitement d'une maladie chez un sujet ;
dans lequel les signaux d'aimantation dynamique de l'étape c) sont mesurés avec un appareil comprenant un magnétomètre CA ; et
dans lequel le signal d'aimantation dynamique est mesuré comme suit :
I. en mesurant les signaux de tension induits en fonction du temps sous des champs magnétiques alternatifs pour :
- les nanoparticules magnétiques fonctionnalisées dans le fluide aqueux ou biologique de l'étape b) contenant l'analyte sous un champ magnétique alternatif ; et/ou pour
- la nanoparticule magnétique fonctionnalisée de l'étape a) d'un échantillon de référence contenant le fluide aqueux ou biologique sans l'analyte sous un champ magnétique alternatif ; et
II. en obtenant la boucle d'hystérésis à partir desdits signaux de tension induits en fonction du temps pour
- les nanoparticules magnétiques fonctionnalisées dans le fluide aqueux ou biologique de l'étape b) contenant l'analyte sous un champ magnétique alternatif ; et/ou pour
- la nanoparticule magnétique fonctionnalisée de l'étape a) d'un échantillon de référence contenant le fluide aqueux ou biologique sans l'analyte sous un champ magnétique alternatif ;
dans lequel la comparaison du signal d'aimantation dynamique de l'étape d) est réalisée en comparant les boucles d'hystérésis ; de préférence en comparant les valeurs des paramètres de boucle d'hystérésis obtenus à partir des boucles d'hystérésis ; et
dans lequel le signal d'aimantation dynamique est mesuré sous des champs magnétiques alternatifs à une seule fréquence de résonance.

10. Utilisation de nanoparticules magnétiques fonctionnalisées pour mesurer l'efficacité d'un traitement d'une maladie chez un sujet, dans laquelle chaque nanoparticule magnétique fonctionnalisée comprend une nanoparticule magnétique et un ligand de reconnaissance, dans laquelle la nanoparticule magnétique a une taille moyenne de 1 à 100 nm et une aimantation à saturation comprise entre 20 et 300 emu/g, dans laquelle le ligand de reconnaissance est lié à ladite nanoparticule magnétique, et dans laquelle l'efficacité dudit traitement est mesurée selon le procédé tel que défini dans la revendication 9.

11. Utilisation selon la revendication 10 pour mesurer l'efficacité d'un traitement d'une maladie, dans laquelle la maladie est sélectionnée parmi sélectionnée parmi le cancer, les maladies auto-immunes, les maladies neurodégénératives, les maladies cardiovasculaires, les maladies inflammatoires et les maladies endocriniennes.

12. Un procédé de diagnostic d'une maladie chez un sujet comprenant les étapes suivantes :
a) la fourniture de nanoparticules magnétiques fonctionnalisées, dans lequel chaque nanoparticule magnétique fonctionnalisée comprend une nanoparticule magnétique et un ligand de reconnaissance, dans lequel la nanoparticule magnétique a une taille moyenne de 1 à 100 nm et une aimantation à saturation comprise entre 20 et 300 emu/g, et dans lequel le ligand de reconnaissance est lié à ladite nanoparticule magnétique,
b) l'incubation des nanoparticules magnétiques fonctionnalisées de l'étape a) avec un fluide aqueux ou biologique provenant du sujet dans des conditions appropriées pour produire la liaison desdites nanoparticules magnétiques fonctionnalisées à un analyte, dans lequel ledit analyte est un biomarqueur de la maladie à diagnostiquer,
c) la mesure du signal d'aimantation dynamique des nanoparticules magnétiques fonctionnalisées dans le fluide aqueux ou biologique provenant du sujet de l'étape b) sous un champ magnétique alternatif, et
d) la comparaison du signal d'aimantation dynamique du fluide aqueux ou biologique du sujet mesuré à l'étape c) à une valeur de référence indiquant la maladie à diagnostiquer ; et
dans lequel les signaux d'aimantation dynamique sont mesurés avec un appareil comprenant un magnétomètre CA ; et
dans lequel le signal d'aimantation dynamique est mesuré comme suit :
I. en mesurant les signaux de tension induits en fonction du temps sous des champs magnétiques alternatifs pour :
- les nanoparticules magnétiques fonctionnalisées dans le fluide aqueux ou biologique de l'étape b) contenant l'analyte sous un champ magnétique alternatif ; et/ou pour
- la nanoparticule magnétique fonctionnalisée de l'étape a) d'un échantillon de référence contenant le fluide aqueux ou biologique sans l'analyte sous un champ magnétique alternatif ; et
II. en obtenant la boucle d'hystérésis à partir desdits signaux de tension induits en fonction du temps pour
- les nanoparticules magnétiques fonctionnalisées dans le fluide aqueux ou biologique de l'étape b) contenant l'analyte sous un champ magnétique alternatif ; et/ou pour
- la nanoparticule magnétique fonctionnalisée de l'étape a) d'un échantillon de référence contenant le fluide aqueux ou biologique sans l'analyte sous un champ magnétique alternatif ; et
dans lequel la comparaison du signal d'aimantation dynamique de l'étape d) est réalisée en comparant les boucles d'hystérésis ; de préférence en comparant les valeurs des paramètres de boucle d'hystérésis obtenus à partir des boucles d'hystérésis ; et
dans lequel le signal d'aimantation dynamique est mesuré sous des champs magnétiques alternatifs à une seule fréquence de résonance.

13. Appareil conçu pour réaliser le procédé défini dans l'une quelconque des revendications 1 à 7, le procédé selon la revendication 9 ou le procédé selon la revendication 12, comprenant un magnétomètre CA pour mesurer le signal d'aimantation dynamique de nanoparticules magnétiques fonctionnalisées comprenant :
a) un générateur de champ magnétique CA configuré pour exciter magnétiquement les nanoparticules magnétiques fonctionnalisées, ledit générateur de champ magnétique CA comprenant une bobine de fil de Litz en tant que bobine d'excitation, dans lequel le générateur de champ magnétique CA fait partie d'un circuit LCR permettant d'injecter de manière résonante un courant CA d'une fréquence de résonance unique dans la bobine de fil de Litz générant un champ magnétique CA dans lequel la fréquence de résonance unique est dans la plage de fréquences de 10 Hz à 1 MHz,
b) un détecteur de flux magnétique comprenant deux bobines exploratrices enroulées en sens inverse reliées en série et montées à l'intérieur de la bobine d'excitation, dans lequel les deux bobines exploratrices présentent les mêmes spires et dimensions, et
c) un lecteur de tension,
dans lequel le lecteur de tension surveille le signal de tension des bobines exploratrices du détecteur de flux magnétique ; et
dans lequel la bobine d'excitation est immergée dans un flux de liquide diélectrique qui circule à travers un échangeur de chaleur pour une thermalisation à température ambiante.

14. Utilisation de l'appareil selon la revendication 13 dans le procédé selon les revendications 1 à 7, dans le procédé selon la revendication 9 ou dans le procédé selon la revendication 12, pour mesurer le signal d'aimantation dynamique des nanoparticules magnétiques fonctionnalisées dispersées dans les fluides aqueux ou biologiques.

15. Utilisation *in vitro* de nanoparticules magnétiques fonctionnalisées pour le diagnostic d'une maladie, dans laquelle chaque nanoparticule magnétique fonctionnalisée comprend une nanoparticule magnétique et un ligand de reconnaissance, dans laquelle la nanoparticule magnétique a une taille moyenne d'environ 1 à environ 100 nm et une aimantation à saturation comprise entre 20 et 300 emu/g, dans laquelle le ligand de reconnaissance est lié à ladite nanoparticule magnétique, et dans laquelle la maladie est diagnostiquée par détection et/ou quantification *in vitro* d'un analyte dans des fluides aqueux ou biologiques selon le procédé tel que défini dans l'une quelconque des revendications 1 à 6 ; et facultativement, dans laquelle la maladie est sélectionnée parmi le cancer, les maladies auto-immunes, les maladies neurodégénératives, les maladies cardiovasculaires, les maladies inflammatoires et les maladies endocriniennes.
